# EUROPEAN PATENT APPLICATION

(11) **EP 1 862 538 A1**
(43) Date of publication of application: **05.12.2007**
(21) Application number: 06114659.3
(22) Date of filing: 29.05.2006
(51) Int. Cl.: C12N 9/12, C12N 15/11

(54) **siRNA kinase and methods of use**

(71) Applicant: IMBA-Institut für Molekulare Biotechnologie GmbH, 1030 Vienna (AT)
(72) Inventor: Martinez, Javier, 1030 Wien (AT); Weitzer, Stefan, 1210 Wien (AT)
(74) Representative: Hammann, Heinz

(57) **Abstract**

The invention is based on the finding that Clp1 has the activity of an RNA kinase. Clp1 is therefore useful as an enhancer of siRNA activity. It can be used per se , in kits or expressed in cell lines. Clp1 transgenic and Clp1 knock-out non-human animals are useful for studying the function of siRNAs. Clp1 is also useful in gene therapy to enhance the efficacy of therapeutic siRNAs.

SEQUENCE LISTING

<110>  IMBA - Institut für Molekulare Biotechnologie GmbH

<120>  siRNA Kinase and Methods of Use

<130>  Case 17-0005

<160>  2

<170>  PatentIn version 3.3

<210>  1
<211>  2437
<212>  DNA
<213>  Homo sapiens

<220>
<221>  5'UTR
<222>  (1)..(723)

<220>
<221>  CDS
<222>  (724)..(2001)

<220>
<221>  3'UTR
<222>  (2002)..(2437)

<400>  1
atgactgact tgtagctgga agaaatcatc ggatttttat tcttttatta aagaaaaaaa      60
atttgaaatg ccttccatgt gccaagcact gtgtcaggtg ggagatgaca gcttggtgaa     120
acctctgtca ggctgtcttc ctccgctttc tctatccctg ggtttccccc tgcctaaaaa     180
ggattttgtg cttcgtggct tgtccaggca agcaggccgt cgcgggacct agaccgagac     240
agtgagtctc tctttctccc gggcctccct tctgtttcct gggctgcagg ggagcaggaa     300
atctggggcg agattcccgc cgcggacgcg cactgccgaa gcctggtccc tcgacctgtc     360
cctgcccagc gcgggggcgc aaccgccacg cctcctcacc cctccctccg gctgcacgaa     420
taatgacaac agccgcccct cccacctttg gcgtcacgtt caaaacaatc ctttgactac     480
aactcccaga aggccgagcg gcttagcgag tgcacccgct ctcggctgct ccggcaaact     540
acacatccca aagggcagcg ccgaccgcgt gtcctttcac agcaaagtgc ggaactgcgt     600
ttgtttccgg cgtgggtccg ggcaagaacc gcttgtagtt tggtttaaat tctgcacggg     660
aggaccttct gagtttacct gttgggctcc tggctgcgca ggcacagcag ctacacagaa     720
gag atg gga gaa gag gct aat gat gac aag aag cca acc act aaa ttt     768
gaa cta gag cga gaa aca gaa ctt cgc ttt gag gtg gag gca tct cag     816
tca gtt cag ttg gag ttg ttg act ggc atg gca gag atc ttt ggc aca     864
gag ctg acc cga aac aag aaa ttc acc ttt gat gct ggt gcc aag gtg     912
gct gtt ttc act tgg cat ggc tgt tct gtg caa ctg agc ggc cgc act     960
gag gtg gct tat gtc tcc aag gac act cct atg ttg ctt tac ctc aac     1008
act cac aca gcc ttg gaa cag atg cgg agg caa gcg gaa aag gaa gaa     1056
gag cga ggt ccc cga gtg atg gta gtg ggc ccc act gat gtg ggc aag     1104
tct aca gtg tgt cgc ctt ctg ctc aac tac gca gtg cgt ttg ggc cgc     1152
cgt ccc act tat gtg gag ctg gat gtg ggc cag ggt tct gtg tcc atc     1200
cct ggt acc atg ggg gcc ctc tac atc gag cgg cct gca gat gtc gaa     1248
gag ggt ttc tct atc cag gcc cct ctg gtg tat cat ttt ggt tcc acc     1296
act cct ggc act aac atc aag ctt tat aat aag att aca tct cgt tta     1344
gca gat gtg ttc aac caa agg tgt gag gtg aac cga agg gca tct gtg     1392
agt ggc tgt gtc att aac acc tgt ggc tgg gtc aag ggc tct ggt tac     1440
cag gct ctg gtg cat gca gcc tca gct ttt gag gtg gat gtc gtt gtt      1488
gtt ctg gat caa gaa cga ctg tac aat gaa ctg aaa cgg gac ctc ccc      1536
cac ttt gta cgc act gtg ctg ctc cct aaa tct ggg ggt gtg gtg gag      1584
cgc tcc aag gac ttc cgg cgg gaa tgt agg gat gag cgt atc cgt gag      1632
tat ttt tat gga ttc cga ggc tgt ttc tat ccc cat gcc ttc aat gtc      1680
aaa ttt tca gat gtg aaa atc tac aaa gtt ggg gca ccc acc atc cca      1728
gac tcc tgt tta cct ttg ggc atg tct caa gag gat aat cag ctc aag      1776
cta gta cct gtc act cct ggg cga gat atg gtg cac cac cta ctg agt      1824
gtt agc act gcc gag ggt aca gag gag aac ctg tcc gag aca agt gta      1872
gct ggc ttc att gtg gtg acc agt gtg gac ctg gag cat cag gtg ttt      1920
act gtt ctg tct cca gcc cct cgc cca ctg cct aag aac ttc ctt ctc      1968
atc atg gat atc cgg ttc atg gat ctg aag tag agatcagcag gaagccttgc    2021
tgcctgggac atagagatca tctggccacc cctagaggca gatgggctga gataaaagac    2081
tgttggggcc acctgaccag taaactgtgg actagtagaa agttcatatt ctacctctaa    2141
aaacaggtag tggtaacctg actcttctaa tcttgaacca aaaggaaaac catgagactg    2201
taattggttt cttagaccac ctaagatgcc actttgaatt ctctaagacc ctggagaatt    2261
gcatttcttt cactgtgcta ctatgtggtt tttaaaaaat caatgcttta tattccatat    2321
gtggttctta cccatttatc atggatgaaa gtgtgaatta gagggactcc ttccaataaa    2381
gttcaaactg aaaaaaaatc attttaataa atatttttgc catatcataa aaaaaa        2437

<210>  2
<211>  425
<212>  PRT
<213>  Homo sapiens

<400>  2

## Description

The present invention relates to the field of molecular biology tools, in particular to the field of nucleic acid phosphorylation.

RNA interference (RNAi) is an evolutionary conserved process in eukaryotes that uses small double-stranded (ds) RNA to specifically silence the expression of cognate target genes [1,2]. RNAi is thought to have originally evolved as a defense system against mobile genetic elements, such as transposons or viruses, which replicate and proliferate in the cell via dsRNA-intermediates, and thereby RNAi acts to maintain genome integrity. RNAi was first formally described only eight years ago by the observation that exogenously introduced dsRNA into *Caenorhabditis elegans* resulted in sequence-specific gene silencing [3]. Since then, the RNAi phenomenon has revolutionized conventional reverse genetic approaches, and is currently widely used as a tool for gene-inactivation studies in a broad range of metazoa, including humans. It further holds great promises as therapeutic technique for the treatment of cancer and other diseases [4].

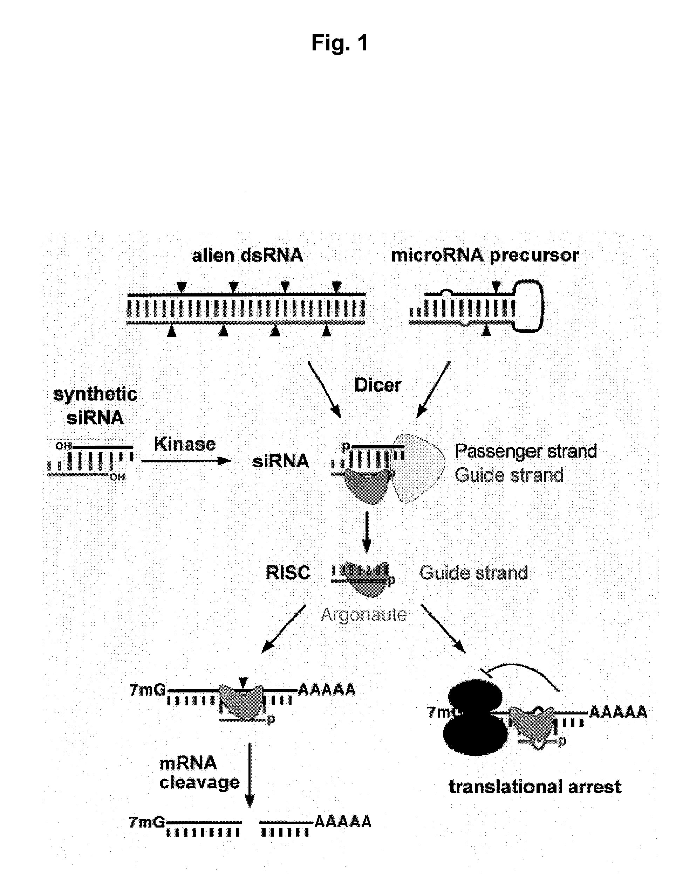

The RNAi pathway is naturally triggered by dsRNA precursors that are processed by the RNase III enzyme Dicer into short duplexes of 21-23 nucleotides (nt) with 3' overhangs of 2 nt (Figure 1) [5, 6]. These duplexes guide the recognition and ultimately the cleavage, and therefore destruction, or translational repression of complementary single-stranded (ss) RNAs such as messenger RNAs (mRNAs). The duplexes are called small interfering RNA (siRNA) if they are perfectly complementary and arise from long dsRNA (for example, from viruses and transposons), and in general guide mRNA cleavage. If the duplexes originate from endogenous noncoding transcripts that form dsRNA with imperfect stem-loop structures, they are termed microRNAs (miRNA) [7]. In animals, miRNAs predominantly inhibit translation by targeting partially complementary sequences in the 3' UTR (untranslated region) of mRNAs. Hitherto more than 1600 miRNAs have been identified in animals, plants and viruses, and it is currently believed that miRNAs are key regulators of gene expression of a vast number of mRNAs and thereby play a major role in the control of development [8].

Gene silencing by both, siRNAs and miRNAs, is initiated by their incorporation into the RNA-induced silencing complex (RISC) [9-11] where the duplexes become unwound in a strand specific manner [12, 13]. Whereas one strand, called the 'passenger' strand, becomes excluded from RISC [14-16], the other strand, called 'guide', stays tightly associated with members of the Argonaute (Ago) family of proteins within RISC and serves as a template for the recognition of the cognate RNA target. Whereas the precise molecular mechanism of translational inhibition by miRNAs has remained unclear, the siRNA guide strand recruits the Ago2 protein to the mRNA target, which is cleaved by the endonucleolytic activity of Ago2 [17, 18].

Processing of dsRNA by Dicer cleavage leaves 5' monophosphates and 3' hydroxyl groups on both, siRNAs and miRNAs. The 5' phosphate on small RNAs is an absolute requirement for efficient RNAi, since siRNA guide strands in which the 5' phosphate was substituted for a 5' methoxy- or amino group did not trigger mRNA target *cleavage in vitro and in vivo* in *Drosophila* as well as in HeLa cells [19-21]. An explanation came from size exclusion chromatography and native gel electrophoresis experiments showing that unphosphorylated siRNA duplexes are not assembled into active RISC [19, 22, 23]. Furthermore, using an *in vitro* system containing recombinant Ago2 and siRNA, it was demonstrated that the 5' phosphate not only is important for RISC assembly, but also plays role within an active RISC itself by contributing to the stability of the Ago2-siRNA complex [18]. It is also a determining factor for the fidelity with which Ago2 cleaves the target RNA [18] that has previously been shown to be set from the 5' end of the guide siRNA [24].

Even though the 5' phosphate is crucial for RNAi, synthetic siRNAs bearing a 5' hydroxyl group can also efficiently mediate RNAi *in vitro* and *in vivo* in *Drosophila* and cultured HeLa cells. This is due to an endogenous 'siRNA-kinase' activity that rapidly phosphorylates the 5' hydroxyl group [19, 21]. So far, the identity of the siRNA-kinase had remained obscure. The purification of a human RNA kinase activity was already attempted over twenty-five years ago, but failed to reveal the protein sequence [25].

It was an object of the invention to elucidate the mechanism of nucleic acid phosphorylation. In particular, it was an object of the invention to identify the human RNA kinase activity that phosphorylates siRNA (the "siRNA kinase").

To solve the problem underlying the invention, a robust biochemical assay was used, which together with conventional chromatography allowed the inventors to successfully purify and identify human Clp1 (HsClp1) as the "siRNA-kinase", the first kinase found in humans that is capable of phosphorylating RNA.

HsClp1 is an evolutionary conserved protein, which was originally identified as a fusion transcript to the AF10 gene after chromosomal translocation and, by its homology to a putative *C.elegans* protein, termed HEAB (human homologue to a hypothetical *Caenorhabditis elegans* ATP/GTP-binding protein) [26]. Clp1 homologues contain a Walker A and possibly a Walker B motif, both of which have been implicated in ATP/GTP binding [27, 28].

Clp1 proteins have previously been shown to be part of the protein machinery that processes precursor mRNA (pre-mRNA). Pre-mRNAs are undergoing various maturing steps prior to their export into the cytoplasm. These include addition of a cap structure at the 5' end, intron splicing and endonucleolytic cleavage at the 3' end followed by polyadenylation. The function of Clp1 was first evaluated in *Saccharomyces cerevisiae* where it was shown to be involved in endonucleolytic cleavage at the 3' end of pre-mRNA [29]. Similarly to yeast, HsClp1 was identified as part of the cleavage factor IIₘ (CF IIₘ) complex that is crucial for 3' cleavage of pre-mRNAs [30]. This factor is part of a multiprotein complex, of which the Cleavage and Polyadenylation Specificity Factor (CPSF) and Cleavage Stimulation Factor (CstF) recognizes specific sequences upstream and downstream of the cleavage site [31, 32]. Other components, such as the cleavage factors CF Iₘ and CF IIₘ are required for the actual cleavage step. Upon cleavage, the enzymatic activity of the poly(A)polymerase, PAP, is responsible for the addition of the poly(A) tail.

Interestingly, the mRNA 3'-end processing machinery has recently been linked to the tRNA splicing pathway in humans. Eukaryotic tRNA introns are usually located in the anticodon loop of the pre-tRNA, and their removal involves multiple enzymatic activities, which have been extensively characterized in *Saccharomyces cerevisiae* [33]. Here, an endonuclease complex composed of the Sen2, Sen15, Sen34 and Sen54 proteins, cleaves the pre-tRNA at the 5' and 3' splice sites, resulting in two tRNA exon half-molecules. The 5' and 3' exons are then ligated by a tRNA ligase, a multifunctional enzyme whose phosphodiesterase, RNA kinase and RNA ligase activities are required to ultimately yield a mature tRNA. By isolating the pre-tRNA endonuclease complex from human cell extracts, the HsClp1 protein together with other components of the mRNA 3'-end processing machine, was found to associate with the HsSen proteins [34]. However, a function of HsClp1 in tRNA splicing remains to be uncovered.

In the experiments of the present invention, it was found that substrates for HsClp1 include dsRNA, dsDNA, dsDNA/RNA hybrids and ssRNA. Interestingly, ssDNA is not phosphorylated by HsClp1, which is a major difference to conventional polynucleotide kinases (PNKs) [35].

The identification of the kinase activity of HsClp1 is the prerequisite for the application of HsClp1 as a tool in molecular biology, e.g. as a research tool for nucleic acid phosphorylation and as a reagent in RNAi techniques, and in therapy where it is useful to enhance the efficiency of gene silencing.

Clp1 homologues are present in almost every organism, even in bacteria and yeast. In addition to the human Clp1, homologues that have the kinase activity of HsClp1, e.g. murine (Mus musculus) Clp1 (AAH03237), Xenopus laevis Clp1 (AAH70530), chicken (Gallus gallus) Clp1 (AJ720420.1), Drosophila melanogaster Clp1 (AE003808.4), Caenorhabditis elegans Clp1 (Z83114.1) are also useful as a research tool and as a reagent in RNAi techniques. In the following, if not stated otherwise, the term "Clp1" is used interchangeably for any mammalian or metazoan homolog that has a kinase activity corresponding to that of the human Clp1, designated HsClp1.

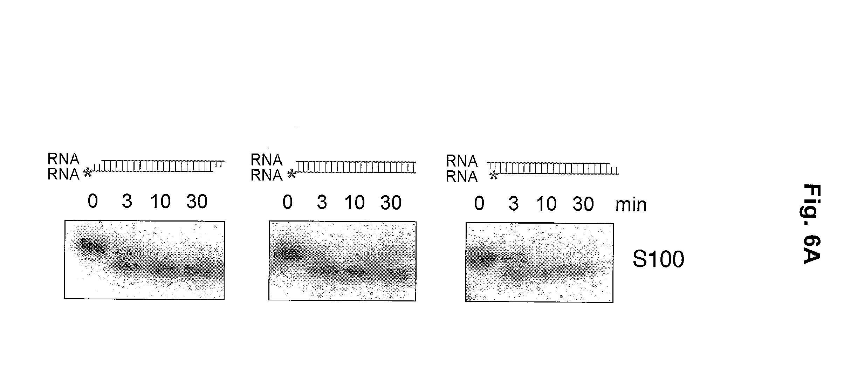

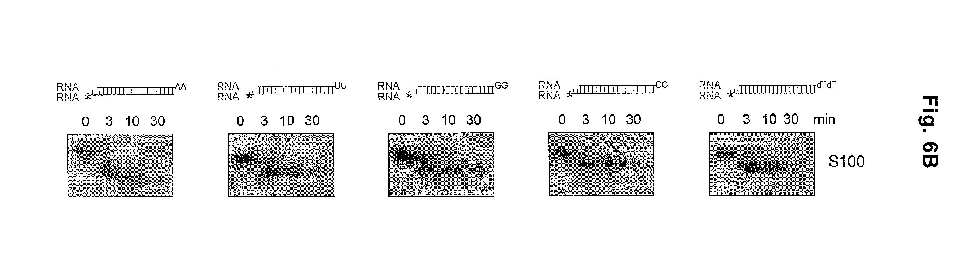

An advantage of Clp1 is that the protein phosphorylates different 5' nucleotides with the same efficiency, at least when present in dsRNA (Figure 6C). In contrast, T4 PNK kinase activity is biased against certain oligonucleotides, especially those with 5'-Cytidine ends [36]. In that respect, Clp1, in particular HsClp1, may be similar to Optikinase (USB corporation), a modified version of T4 PNK, which also shows little or no discrimination against different oligonucleotides. However, there is a major difference between all types of T4 PNKs (the naturally occurring one and the modified Optikinase) on the one hand and HsClp1 on the other hand, i.e. the ability to discriminate between ssRNA and ssDNA, the latter being not phosphorylated, which is of importance to address specific questions in molecular biology approaches.

In the meaning of the present invention, the use of the term "kinase activity" in the context of Clp1 refers to the above-mentioned activity of Clp1 to phosphorylate dsRNA, dsDNA, dsDNA/RNA hybrids and ssRNA.

Thus, in a first aspect, the present invention relates to the use of Clp1 for the transfer of the γ-phosphate of ATP to the 5' end of ssRNA, dsRNA or the 5'end of a RNA strand as part of RNA/DNA hybrids.

In a preferred aspect, the invention relates to the use of recombinant Clp1.

In a further aspect, the invention relates to the use of recombinant HsClp1.

HsClp1 has the amino acid sequence as depicted in SEQ ID NO:2, it is encoded by a DNA sequence as depicted in SEQ ID NO:1.

Recombinant HsClp1 (or, in an analogous manner, any mammalian or metazoan homolog of HsClp1, can be readily obtained by expression of genetic constructs comprising one or more Clp1 DNA sequences operably linked to regulatory DNA sequences (which may be heterologous regulatory sequences), such as promoters or enhancers, in host cells, preferably in bacterial, fungal (including yeast), plant or animal (including insect or mammalian) cells. In such constructs, the design of which is described in laboratory manuals (see e.g. [37]) and is routine to the skilled artisan, the regulatory sequences may be operably linked to a polynucleotide encoding mature Clp1 polypeptide or a variant thereof. To obtain Clp1 polypeptides useful for the present invention, besides the DNA molecules having a nucleotide sequence corresponding to the naturally occurring sequence, DNA molecules with a substantially different sequence may be used, which, due to the degeneracy of the genetic code, still encode a Clp1 polypeptide with the desired kinase activity. Since the genetic code is well known in the art, it is routine for one of ordinary skill in the art to produce the degenerate variants described above without undue experimentation.

Thus, Clp1 polypeptides also encompass variants which have deviations from those encoded by the naturally occurring DNA molecules (the sequence of which, in the case of HsClp1, is depicted in SEQ ID NO:1). Such deviations may be caused by the conservative exchange of amino acids, as long as they are Clp1 derivatives or fragments or peptides with the desired kinase activity, accordingly, isolated DNA molecules encoding such derivatives or fragments with a polynucleotide sequence varying in their sequence from the naturally occurring DNA sequence may be used for expression in host cells to produce the recombinant Clp1. A variant or fragment Clp1 candidate sequence may be routinely tested for its kinase activity in an assay as described in the experiments of the invention.

The sequence encoding the Clp1 polypeptide may be fused to a marker sequence, such as a sequence encoding a peptide which facilitates purification of the fused polypeptide. The marker amino acid sequence may be a hexa-histidine peptide, such as the tag provided in a pQE vector (Qiagen, Inc.), among other tags, many of which are commercially available, like the "HA" tag, another peptide useful for purification, which corresponds to an epitope derived from the influenza hemagglutinin protein. Yet another useful marker peptide for facilitation of purification of Clp1 is glutathione S-transferase (GST) encoded by the pGEX fusion vector (see, e.g. [38]). Other such fusion proteins include Clp1 fused to immunoglobulin Fc at the N- or C-terminus.

In a preferred aspect, Clp1 is the human HsClp1 polypeptide with the amino acid sequence as set forth in SEQ ID N0:2 or with the amino acid sequence encoded by a polynucleotide which hybridizes under stringent conditions to a polynucleotide having a nucleotide sequence as set forth in SEQ ID NO:1 or the region encoding hsClp1 contained therein (nucleotides 724 to 2001).

According to the present invention, Clp1 may be provided *per se.* Alternatively, Clp 1 may be used as a component of a kit.

Thus, in a further aspect, the invention relates to a kit that contains Clp1 in combination with the reagents that ensure its optimal kinase activity. In this embodiment of the invention, the kit contains
a) a recombinant Clp1;
b) γ-ATP; and
c) a reaction buffer containing one or more metal ions selected from Mg²⁺, Mn²⁺, Ni² or mixtures thereof, for use in a final concentration range of ca. 1-10 mM, preferably a concentration range of 2 -5 mM.

Besides the above-mentioned metal ions, the buffer of the kit contains the usual buffer components that are known to the person skilled in the art; these components may be present in a combination as used in the experiments of the present invention (KCl, Hepes pH 7.4, glycerol, DTT, AEBSF (4-(2-aminoethyl)benzenesulfonyl fluoride) or they may be similar or identical to the reaction buffer used for T4 PNK reactions, e.g. 70 mM Tris-HCl, 10 mM MgCl₂, 5 mM dithiothreitol (DTT). Another possible other buffer system that has been successfully tested in kinase assays is 50 mM KH₂PO₄/K₂HPO₄ pH 7.2. In general, the kinase is active in buffers ranging from pH 6.0 (50 mM MES, Morpholineethanesulfonic acid) up to pH 9.0 (50 mM CHES, N-Cyclohexyl-2-aminoethanesulfonic acid) and is active at salt concentrations ranging from 10 - 200mM KCl (see Example 5).

If the kit of the present invention is used for radioactively labeling nucleic acids, γ-ATP may be present in radioactively labeled form, e.g. [γ-³²P]ATP, [γ-³³P]ATP, [γ-¹⁸O]ATP or [γ-³⁵S]ATP.

Preferably, the Clp1 contained in the kit is HsClp1.

Recombinant Clp1, or a kit containing it, is *inter alia,* useful for all types of reactions as commonly used for the preparation of radioactive RNA probes to monitor RNA processing or for hybridizations (such as Northern Blotting). Phosphorylated nucleic acids, e.g. siRNA molecules, that have been obtained by using Clp1 as the active kinase, are e.g. useful in experiments that aim at determining the efficiency of knock-down experiments. By using phosphorylated in admixture with varying proportions of unphosphorylated siRNA molecules, the degree of phosphorylation that is optimal for the desired knock-down effect can be determined.

Previous studies have investigated the requirement of a potential 'siRNA-kinase' for phosphorylation of synthetic exogenous siRNAs that contain 5'-hydroxyl groups for entering the RNAi pathway [19-21]. Clp1 may also have a role in maintaining 5'-phosphate groups on siRNA duplexes. This role is of physiological relevance as the RNase III enzyme Dicer generates 5' phosphorylated endogenous siRNAs and miRNAs by cleavage from long dsRNAs or precursor-miRNAs, respectively. To keep the 5'-phosphate at steady-state, Clp1 may have to overcome putative phosphatase activities that readily dephosphorylates RNA duplexes. Indeed, it has been reported that *Drosophila* embryo lysates contain such a siRNA-phosphatase activity [19].

A human 'siRNA-phosphatase' (the existence of which may be expected with high likelihood due to the high degree of conservation of these mechanism between species), could reduce the rate of the cellular siRNA-kinase activity of Clp1. This could become a rate-limiting step for efficient endogenous RNAi as well as when using RNAi as laboratory tool to knock-down a gene of interest. It has been shown that the efficiency of transient gene silencing in mammalian tissue culture by using RNAi varies, a disadvantage that has mostly been attributed to the sequence chosen for siRNA design, the efficiency of cell transfection and protein stability. So far, the cellular stability of the 5'-phosphate of siRNAs has been neglected as a factor contributing to successful RNAi.
Therefore, an increase of the cellular level of Clp1, for example by overexpression of Clp1 in mammalian cells, is desirable to improve the effectiveness of RNAi, when using siRNAs (or vectors encoding short-hairpin RNAs [shRNAs]). Controlled cellular levels of Clp1 can also be provided by generating stable mammalian cell lines expressing the enzyme, which could be transfected with siRNAs (or vectors encoding for short-hairpin RNAs [shRNAs]) against the gene of interest.

Thus, in a further embodiment, the present invention relates to a cell line genetically engineered to express Clp1, preferably a human cell line that stably expresses HsClp1. Stable expression is achieved by inserting the Clp1 DNA, under the control of a promoter, preferably an inducible promoter, into the genome of the cell, which can be achieved by conventional methods that are commercially available and/or described in the literature, e.g. the tetracycline-inducible system such as the T-REx System (Invitrogen), or the estrogen receptor inducible system [39]. Such cell lines are useful in combination with siRNA vectors, so-called "hairpin" vectors that are commercially available (e.g. the pSUPERIOR vector system, Oligoengine), for studying the effect of a knock-down of a gene of interest in the cell line. There are no restrictions with regard to the cell line, it may be a tumor cell line (MCF7, MDA-MB-468, PC-3, HeLa,

SAOS-2, U2OS, A172, U87MG, Colo5, HCT116, NCI-H460, an immortalised primary cell line, e.g. a fibroblast cell line like BJ1-hTERT, a mammary gland epithelial cell line , a keratinocyte cell line, a T or B cell line, a mast cell line, a macrophage cell line, and other haematopoietic cells lines, a neuronal cell line, a liver cell line or a cell line derived from any other organ or tissue, or primary cells such as freshly isolated T cells or cardiomyocytes. There are numerous commercially available cell lines, e.g. from ATCC (American Type Culture Collection; P.O. Box 1549 Manassas, VA 20108, USA) or DMSZ (Deutsche Sammlung von Mikroorganismen und Zellkulturen GmbH, Mascheroder Weg 1b, 38124 Braunschweig, GERMANY).

Alternatively, the genetically engineered cell line does not express Clp1 from a DNA sequence that has been integrated in its genome, but transiently expresses Clp1 from a conventional vector (e.g. the pcDNA system from Invitrogen), carrying the Clp1 sequence under the control of a promoter, preferably an inducible promoter, as described above (e.g. a tetracycline-inducible system).

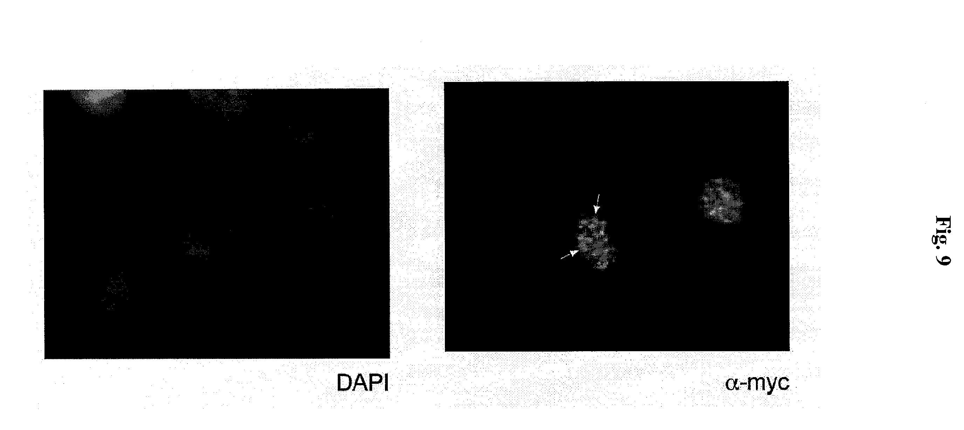

In another embodiment, the Clp1 DNA sequence (either inserted into the genome or present on a vector), is fused to a sequence encoding a cytoplasmatic localization signal, e.g. myristylation signal, for example the src-protein myristylation membrane localization signal (N-term.-MGSSKSKPKDPSQRR-C-term.). As shown in Figure 9, the predominant localization of overexpressed HsClp1 is in the nucleus, where it is present in protein complexes involved in 3'-end processing of pre-mRNAs and together with tRNA splicing endonucleases. To avoid interference with the nuclear functions of endogenous Clp1, it is desirable, in the case of cell lines that endogenously express Clp1, to have the overexpressed Clp1 artificially retained in the cytoplasm. Since the function of Clp1 to phosphorylate siRNAs appears to occur in the cytoplasm, cytoplasmic localization potentiates the function of Clp1, thereby enhancing RNAi. Thus, cytoplasmic localization as achieved by fusing Clp1 to a cytoplasmatic localization signal, i.e. a myristylation signal, results in anchoring the Clp1 protein in the cytoplasmic membrane, may be advantageous in all the above described applications of Clp1 to improve RNAi.

The siRNA molecule encoded by the hairpin vector may also be under the control of an inducible promoter, preferably the same one as the one controlling expression of Clp1. This provides the advantage that expression of Clp1 and the siRNA molecule can be induced simultaneously, by adding only one inducer.

In a further embodiment, the cells genetically engineered to express Clp1 is a cell line that does not endogenously express Clp1.

Such cells can be obtained by generating, in a first step, a Clp1 knock-out cell line, i.e. a cell line that does not express Clp1. Such a cell line can be obtained by conventional methods, e.g. a method that has been described for targeted transgene insertion into human chromosomes by adeno-associated virus vectors and used for generating human somatic cell gene knockouts [40-42]. Moreover, such cells can be generated from mouse ES cells and knock-out mutant mice where both copies of Clp1 are disrupted either by conventional gene targeting or using tissue-specific gene targeting strategies using floxed alleles [43]. Floxed alleles allow to delete Clp1 in defined tissues of mouse embryos and adult mice as well to delete Clp1 in cells derived from these mice using transfection with Cre containing vectors, e.g. adenomCre viruses to delete the floxed Clp1 gene in keratinocytes, fibroblasts or any other cell derived form such an approach.

The invention further relates to a Clp1 knock-out mouse. In yet another embodiment, the invention relates to a knock-out cell line.

A typical approach for generating a Clp1 knock-out mouse and Clp1 knock-out cell lines applies a conventional method that uses gene targeting by homologous recombination to insert artificial DNA into chromosomal DNA contained in mouse embryonic stem cells (ES cells), is described in Example 7.

Clp1 knock-out cell line can be genetically engineered to stably express Clp1 from its genome or from a vector, as described above. A cell line that does not endogenously express Clp1 provides the advantage that siRNA knock-down experiments can be conducted in a precisely controlled way.

In a further aspect, the present invention relates to a Clp1 transgenic non-human animal, which is useful for RNAi-mediated gene function studies.

In an embodiment, the non-human animal is a mouse. Conventional and inducible, tissue specific knock-out mice as well as transgenic mice expressing wild type or constitutively active Clp1 are valuable tools for improved testing of the *in vivo* function of siRNAs directed against a target gene of interest. By way of example, the function of a novel siRNA, e.g. for a novel target gene, can be tested in a transgenic mouse expressing Clp1, e.g. under an inducible promotor, with respect to efficacy or side effects that result from deletion of the entire protein encoded by the target gene. Such information provides the basis for the idenfication and description of the maximum effects that might be due to the inhibition of the target gene. Such system can also be used to identify so-called "off-target effects" (i.e. effects of a compound that are not target-related, in particular undesired toxic effects). Thus, the information obtainable from the transgenic Clp1 mouse treated with siRNA directed to a specific target gene is valuable in view of lead optimization and profiling of candidate small molecule inhibitors of a given target gene that have been identified in a drug screening process. On the other hand, it can be tested whether an siRNA molecule does not work in Clp1 knock-out mice, which provides a very useful control of specificity..

In a further embodiment, the non-human animal is a metazoan. In further embodiment, the metazoan is *C.elegans.* To obtain *C.elegans* Clp1 transgenes, general protocols such as described in Praitis et al. can be followed [44]. In yet another embodiment, the metazoan is *Drosophila melanogaster.* Clp1 transgenic *Drosophila can* be obtained by P-element vector transformation as described in [45, 46].
In another embodiment, the present invention relates to the use of a DNA molecule encoding HsClp1 (coding region of SEQ ID NO.1) or a fragment or derivative thereof, the expression product of which exhibits the kinase activity of HsClp1, as defined above, in RNAi-based gene therapy approaches. In this embodiment, HsClp1 is expressed jointly with therapeutic siRNA molecules, either from a single vector or independently from siRNA vectors.
In the following citations [47, 48], vector-based systems for stable expression of short interfering RNAs are reported. These systems are based on a vector, in which a synthetic, gene-specific target sequence encoding the siRNA is expressed under the control of a promoter that is suitable for transcription of small, non-coding RNA. The siRNAs are thus produced from the vector following its introduction into mammalian cells by standard transfection protocols, e.g. electroporation, lipofection. Most commonly used vectors are adenoviral, adeno-asssociated or lentiviral vectors. An example for RNAi-mediated gene silencing *in vivo* is given by [49]; a review of siRNA therapeutics by [50]. There is no restriction with respect to the target; depending on the disease, any target may be envisaged for the design of inhibiting siRNA molecules, e.g. the EGFR in breast cancer, BCR-Abl in CML, TNFalpha in rheumatoid arthritis, PKC theta in T cells, B-Raf in melanoma, AKT or PI3K in various PTEN-deficient tumors, myotubularins in neuromuscular diseases like X-linked myotubular myopathy. However, any disease-causing gene and any cell type or tissue can potentially be targeted. The use of Clp1 DNA in combination with therapeutic siRNA is particularly useful for targeting disease relevant mutated alleles. In this approach, siRNA designed to inhibit a specific mutation is combined with hClp1 DNA on a vector, which, when containing a tissue-specific promotor, allows for tissue-specific expression of the therapeutic siRNA. Examples for such application is the EGFR mutation at aa position 858 in non-small cell lung cancer or BCR-Abl.

In yet another aspect, the present invention relates to a method for determining whether a test compound is an agonist of Clp1, in particular HsClp1.

Such assays can be conducted in specialized kinase assay formats with dsRNA, dsDNA, dsDNA/RNA hybrids and ssRNA as a substrate for Clp1 and using the buffer and other reagents as described above. 1. When such kinase assay is in the form of a screening assay, as described in Example 8, small molecules can be identified that are activators or inhibitors of Clp1 to enhance or decrease siRNA and microRNA processing via Clp1. Compounds identified as enhancers of Clp1 function can be used in combination or adjuvant therapy with siRNA molecules. Compounds enthancing Clp1 activity can be also employed to enhance the efficacy of RNAi or micro RNA processing in experimental settings (e.g. large scale screenings for gene function by use of RNAi-libraries) or in therapeutic settings where the efficacy of siRNA molecules or micro RNA molecules employed as drugs can be modified by enhancing (or decreasing) Clp1 function.

In summary, Clp1 is useful to improve the sensitivity of whole organisms (e.g. mice) or cell lines for testing the efficacy of RNAi or for general large scale screenings in different/primary mammalian cells, which is the basis for developing siRNA/microRNA with modified efficacy into drugs

### Brief description of the Figures:

Figure 1: Schematic representation of the RNAi pathway.
Figure 2: Assay to measure siRNA kinase activity.
   The guide strand of a siRNA duplex is radiolabeled at the 3' end and annealed to a complementary RNA oligonucleotide. The siRNA duplex served as substrate in a reaction mixture containing ATP and Mg . Phosphorylation of the 5'-end of the guide strand after incubating with kinase-active fractions (such as HeLa S100 extract) is detected by separating the reaction products on a 15% denaturing sequencing gel, followed by Phosphoimaging.

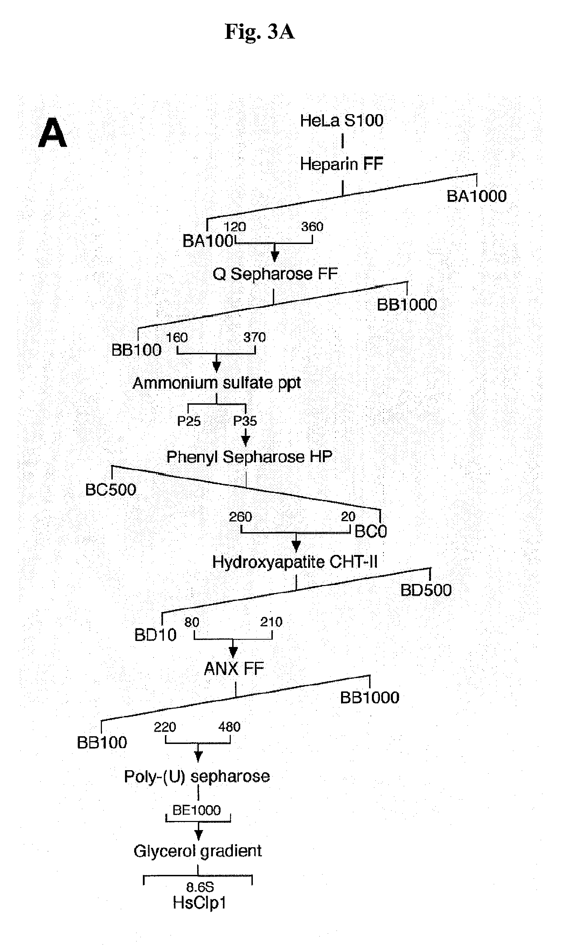

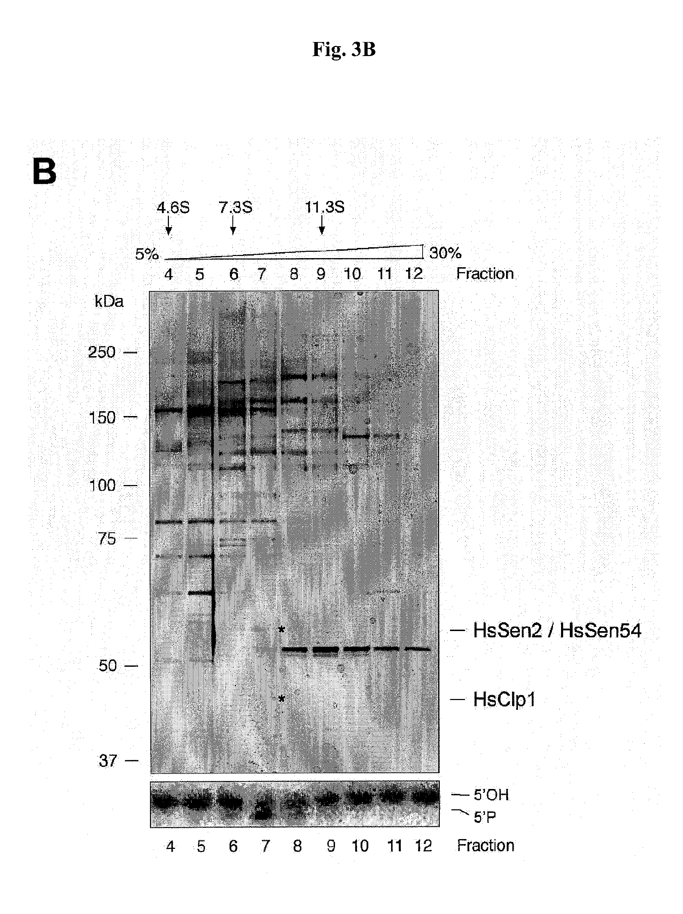

Figure 3: Purification and identification HsClp1 as a human 'siRNA kinase'.
   A, Purification scheme to isolate the siRNA-kinase activity from HeLa S100 extracts. B, Fractions obtained at the final purification step (5% - 30% glycerol gradient) are run on a 7.5% SDS-PAGE and subjected to silver staining. An activity assay on the fractions is performed in parallel (lower panel). The silver-stained band corresponding to a 45 kDa-protein, that co-purifies with the activity (fraction 7), is indicated by an asterisk.
   C, Activity assay after size exclusion chromatography (Superdex 200) on a semi-purified kinase fraction. The peak activity is detected in fractions 20-24.

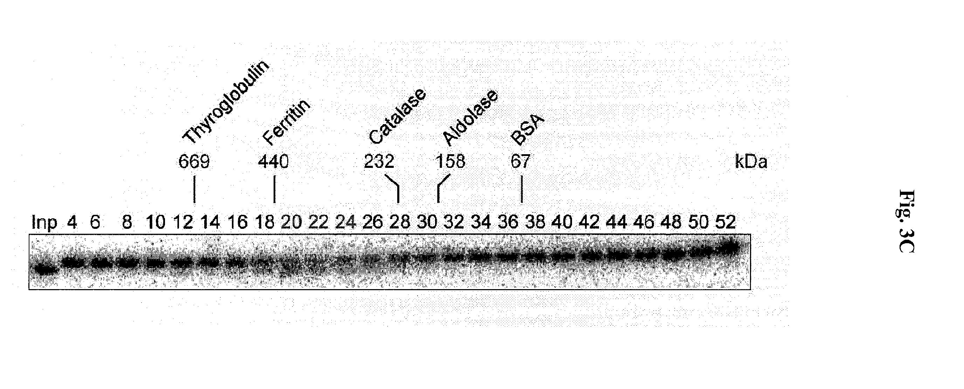

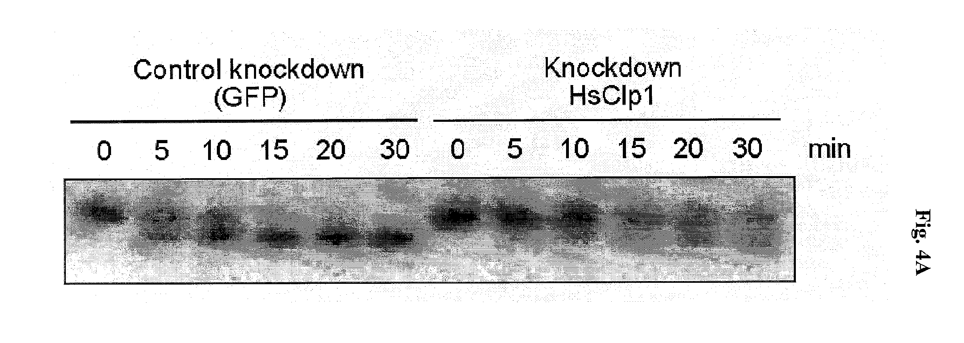

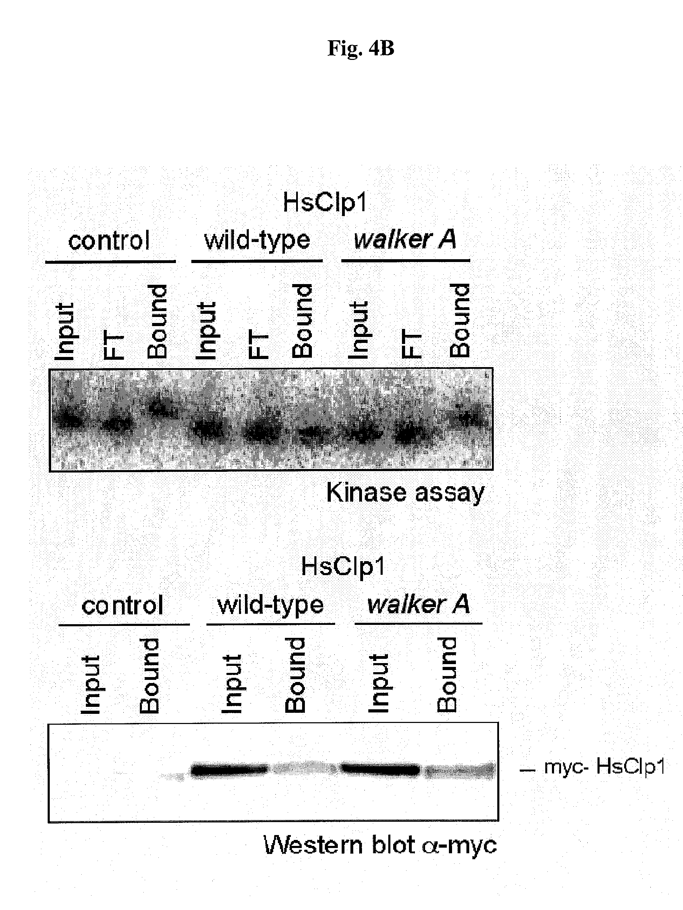

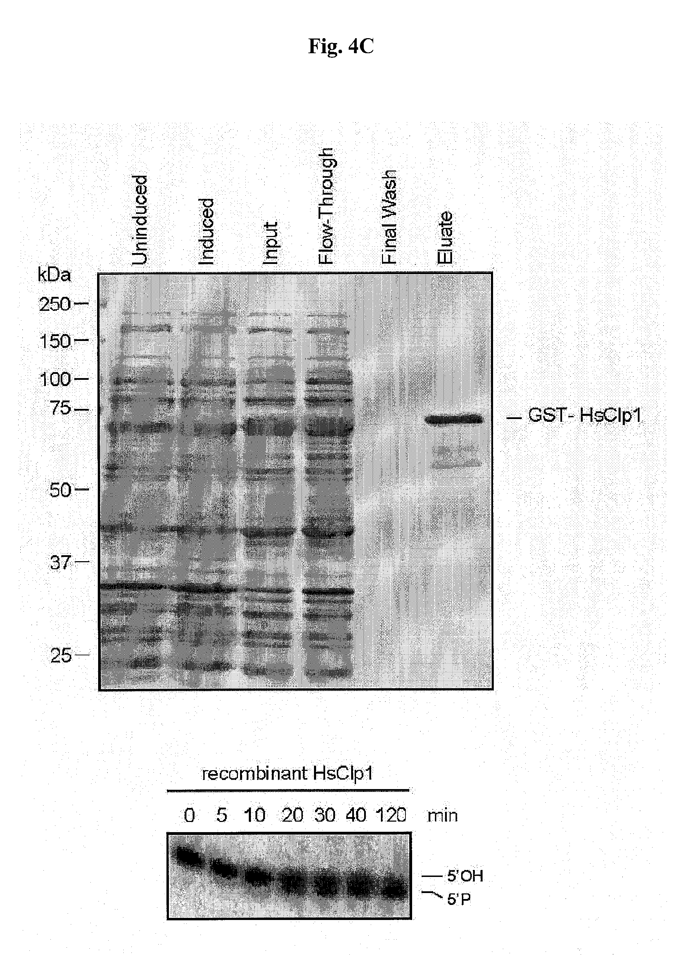

Figure 4: Validation of the identity of HsClp1 protein as the 'siRNA kinase'.
   A, HeLa cells are transfected with siRNA complementary to HsClp1 or with a GFP control-siRNA. Cell extracts are prepared, protein concentrations equalized, and the extracts assayed for kinase activity.
   B, HeLa cells are either transfected with an expression vector encoding the myc-tagged HsClp1 protein (wt) or a myc-tagged version of HsClp1 containing a point-mutation in the Walker A-site, or left untransfected (control). Immunoprecipitates of the extracts using α-myc antisera are tested for kinase activity (upper panel) or are subjected to Western Blotting to confirm the presence of myc-tagged proteins (lower panel).
   C, The recombinant HsClp1 protein is produced as glutathione S-transferase fusion by expression in *E.coli* and purified using glutathione sepharose. Aliquots obtained during the purification are analyzed by Coomassie staining (upper panel), and the eluate fraction containing HsClp1 is assayed for kinase activity (lower panel).

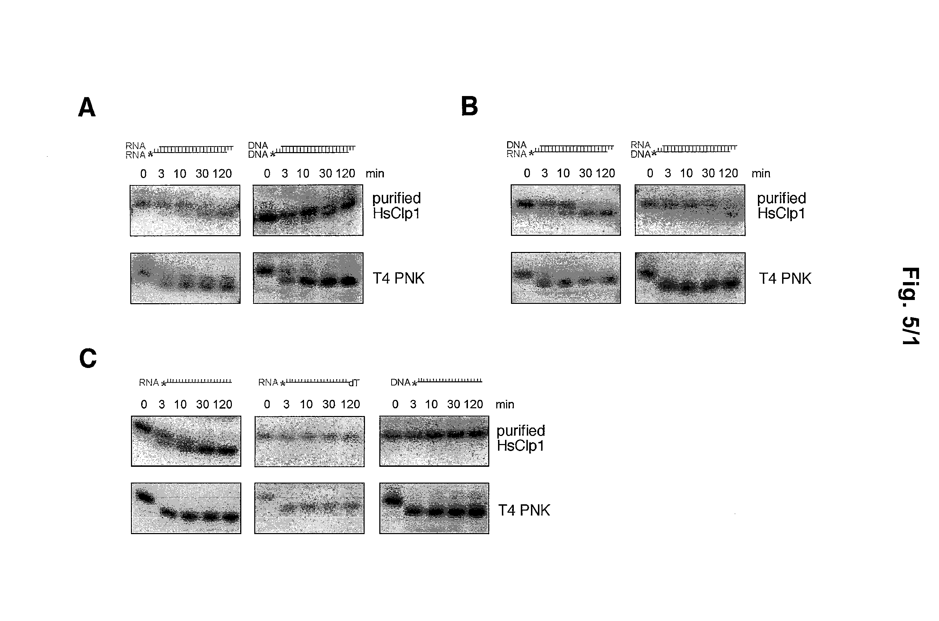

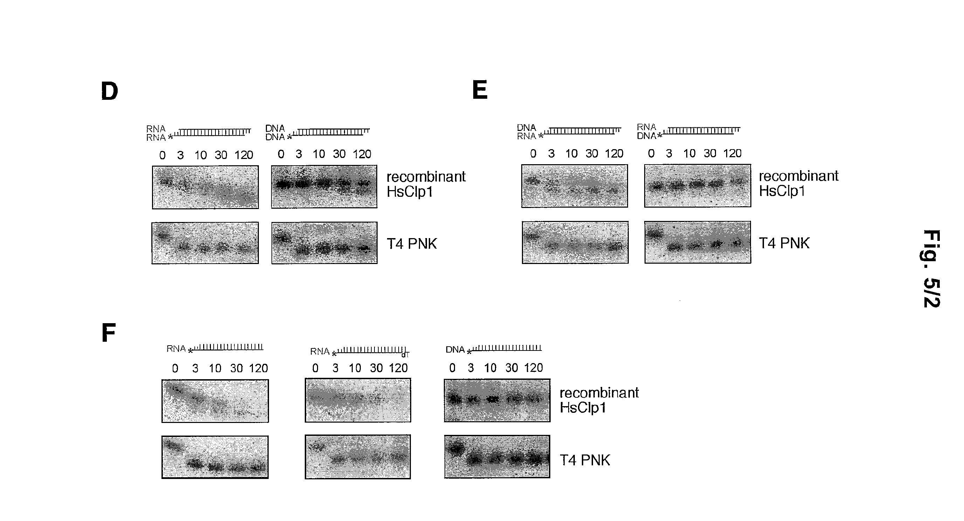

Figure 5: HsClp1 discriminates between ssRNA and ssDNA.
   'Endogenous' HsClp1 purified from HeLa extracts (A - C) or recombinant HsClp1 (D - F) are tested for their ability to phosphorylate the indicated double-stranded and single-stranded oligonucleotide substrates. The substrate specificity of T4 polynucleotide kinase (New England Biolabs) is tested in parallel reactions. Asterisks indicate the position of the radiolabel.
Figure 6: Substrate specificity of HsClp1 at dsRNA termini.
   The kinase activity of the HsClp1 is independent of the type of overhang (A), the nucleotide composition of the overhang (B) and the nucleotide at the 5'-end of a siRNA (C). HeLa S100 extract is incubated with the indicated siRNA duplexes followed by a kinase activity assay. Asterisks indicate the position of the radiolabel.

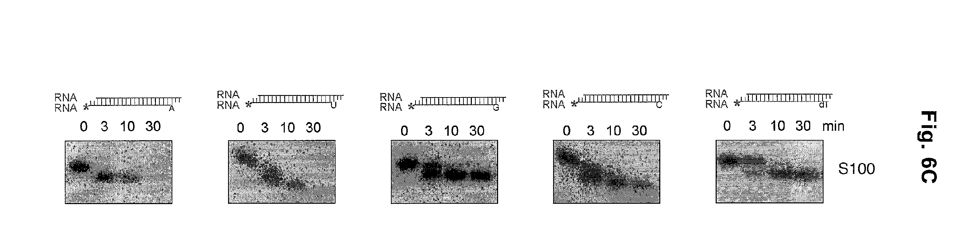

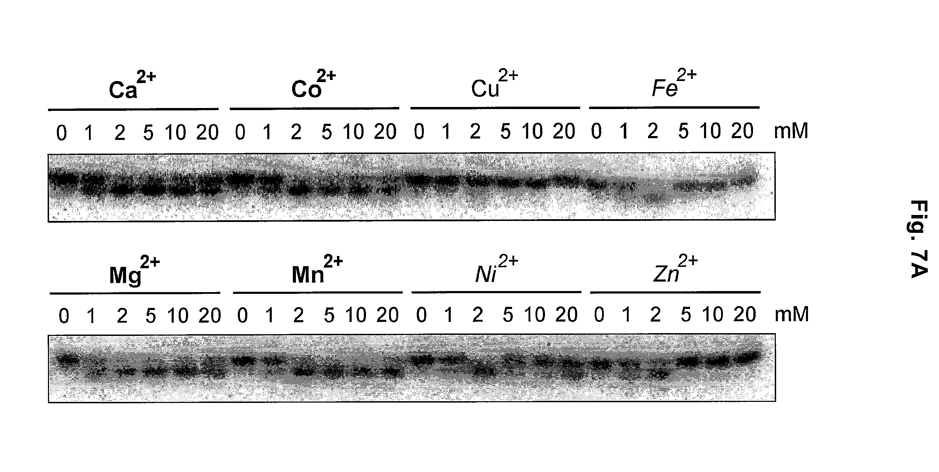

Figure 7: Requirement of divalent metal-ions for the kinase activity of HsClp1.
   A semi-purified 'endogenous' HsClp1 fraction from HeLa extracts (active pool from the Q sepharose chromatography [see Figure 3A])(A), and the recombinant HsClp1 (B) is dialyzed against buffer lacking metal ions, treated with EDTA and dialyzed again against buffer devoid of metal ions. The kinase activity is assayed in a reaction mixture supplemented with divalent-metal ions at the indicated concentrations. The different fonts indicate reactions where kinase activity is high (bold), only show a narrow window of activity *(italics)* or is absent (regular).

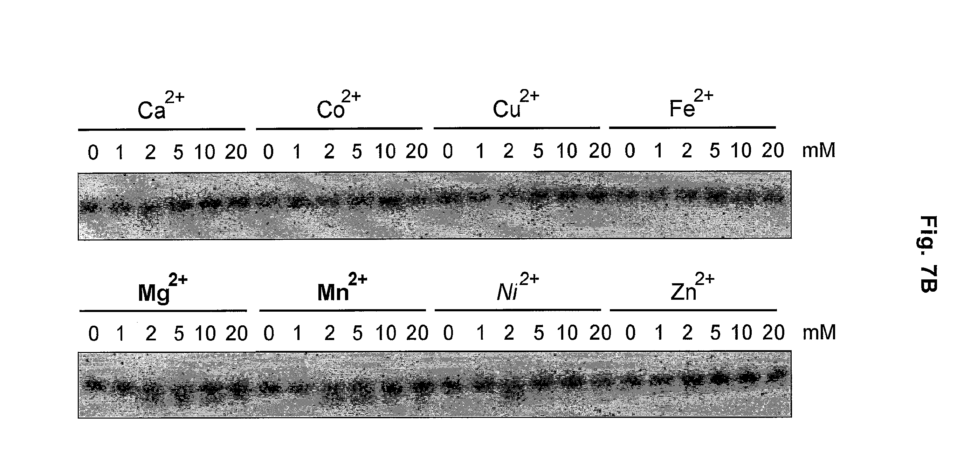

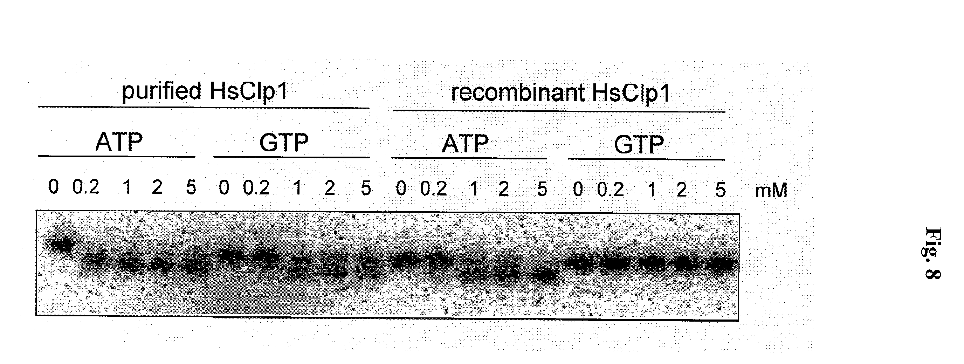

Figure 8: Requirement of ATP and GTP for the kinase activity of HsClp1.
   The kinase activity of a semi-purified 'endogenous' HsClp1 from HeLa extracts (active pool from the Q sepharose chromatography [see Figure 3A]), and the recombinant HsClp1 is tested in reaction mixtures containing the indicated concentrations of ATP and GTP.
Figure 9: HsClp1 localizes predominantly to the nucleus.
   HeLa cells are transfected with an expression vector encoding a myc-tagged version of the HsClp1 protein. Cells are fixed with para-formaldehyde and immunostained using polyclonal α-myc antiserum (right). Nuclei are visualized with DAPI (left).
   In the Examples, if not otherwise stated, the following materials and methods are used.

### 1. Assay to detect siRNA-kinase activity in human extracts

An RNA oligonucleotide derived from the firefly luciferase gene (5'- UCGAAGUAUUCCGCGUACGU -3', guide strand) is chemically synthesized (Dharmacon) and subjected to a 3' Terminal ³²pCp labeling reaction. The labeling is performed in a 20 µl reaction (100 pmol RNA oligonucleotide, 3.3 µM Cytidine 3', 5'-bis [α-³²P] phosphate (GE healthcare), 15% DMSO, 40 U T4 RNA ligase (New England Biolabs, NEB), and 1x NEB-supplied reaction buffer) for 1 h at 37°C. The labeled RNA is gel-purified, ethanol-precipitated and dephosphorylated (120 µl reaction, 1 U Alkaline Phosphatase [Roche], and Roche-supplied buffer) for 30 min at 37°C. The reaction is then deproteinized by Proteinase K, followed by phenol/chloroform extraction and ethanol precipitation. 10 pmole of the labeled RNA are then annealed to 10 pmole of a complementary oligonucleotide (5'- CGUACGCGGAAUACUUCGAAA -3', Dharmacon) in 200 µl reaction buffer (100 mM KCl, 5 mM MgCl₂, 10% glycerol, 0.5 mM DTT, 0.1 mM AEBSF), at 90°C for 1 min, followed by incubation at 37°C for 1 h, resulting in a 50 nM siRNA duplex. For DNA labeling, 4 pmol of a DNA oligonucleotide corresponding to the firefly luciferase sequence described above is incubated in a reaction mixture containing 24 units Terminal Deoxynucleotidyl Transferase (Promega), 1x Promega-supplied reaction buffer and 0.5 µM [α-³²P] cordycepin-5'-triphosphate (Perkin Elmer) at 37°C for 30 min. The labeled DNA is gel-purified, ethanol precipitated and dissolved in H₂O to a final concentration of 20 nM. Annealing with RNA or DNA oligonucleotides is performed as described above.

The activity assay is performed by adding 2.5 µl of the sample of interest to 2.5 µl of a reaction mixture R (100 mM KCl, 5 mM MgCl₂, 10 mM DTT, 2 mM ATP, 0.4 mM GTP and RNasin [Promega]) containing 5 nM labeled siRNA, followed by incubation at 30°C. The kinase reaction is stopped by adding 5 µl of 8 M urea solution. Reaction products are separated on a 15% denaturing acrylamide gel, and siRNA phosphorylation is monitored by Phosphorimaging.

### 2. Purification of the siRNA-kinase activity from human extracts

### 2.1. Preparation of S100 extracts from HeLa cells

Cytoplasmic extract from HeLa cells is prepared according to the Dignam protocol, designed for the isolation of HeLa cell nuclei [51]. The cytoplasmic fraction is supplemented to final concentrations of 100 mM KCl, 2 mM MgCl₂ and 10% glycerol, quick-frozen in liquid nitrogen and stored at -70°C. S100 extracts are prepared by ultracentrifugation for 1 h at 29000 rpm at 4°C using a Sorvall T-1250 rotor. The protein concentration of HeLa S100 extracts is usually 3-5 mg/ml.

### 2.2. Purification procedure

The purification of HsClp1 as the siRNA-kinase activity is schematically shown in Figure 3A. All procedures are carried out at 4°C. Column fractions are assayed for siRNA-kinase activity as described above. 500 ml HeLa S100 extracts (4 mg/ml) are loaded onto a Heparin Sepharose 6 FF column (XK26, 100 ml bed volume, GE Healthcare) equilibrated in buffer BA100 (100 mM KCl, 30 mM Hepes pH 7.4, 5 mM MgCl₂, 10% glycerol, 0.5 mM DTT, 0.1 mM AEBSF). Protein is eluted over 5x CV (column volumes) over a linear gradient in buffer BA1000 containing 1M KCl. The 120 mM-360 mM fraction (160 ml, 260 mg of protein) are dialyzed overnight against buffer BB100 (100 mM KC1, 30 mM Tris pH 8.0, 5 mM MgCl₂, 10% glycerol, 0.5 mM DTT, 0.1 mM AEBSF) and applied to a HiTrap Q sepharose FF column (20 ml bed volume, GE Healthcare) equilibrated in buffer BB100. The column is developed with a gradient over 15x CV to buffer BB1000 containing 1M KC1. The active pool (160-370 mM, 60 ml, 70 mg of protein) is supplemented with 25% ammonium sulfate (8.8 g), left for 15 min on ice, and spun for 20 min at 16000 rpm at 4°C using a Sorvall SS34 rotor. 3.8 g ammonium sulfate (35%) is added to the supernatant, the solution is again left for 15 min on ice and spun for 20 min at 16000 rpm at 4°C. One half of the pellet (10 mg of protein) is dissolved in buffer BC500 (500 mM (NH₄)₂SO₄, 100 mM KC1, 30 mM Hepes pH 7.4, 5 mM MgCl₂, 10% glycerol, 0.5 mM DTT, 0.1 mM AEBSF) and loaded onto a HiTrap Phenyl Sepharose HP column (5 ml bed volume, GE healthcare) equilibrated with buffer BC500. Protein is eluted with a gradient over 12x CV to buffer BC0 (lacking (NH₄)₂SO₄). The active pool (260-20 mM (NH₄)₂SO_{4,} protein amount beyond measurability) is dialyzed overnight against buffer BD10 (10 mM KPᵢ pH 7.2, 50 mM KCl, 10% glycerol, 0.5 mM DTT, 0.1 mM AEBSF) and applied to a Hydroxyapatite CHT-II column (5 ml bed volume, Biorad) equilibrated with buffer BD10. The column is developed with a gradient over 12x CV to buffer BD500 (containing 500 mM KPᵢ). Fractions (80-210 mM) are pooled, dialyzed overnight against buffer BB100 and loaded onto a HiTrap ANX FF column (1 ml bed volume, GE healthcare) equilibrated with buffer BB100. Protein is eluted with a gradient over 12x CV to buffer BB1000. The active pool (220-480 mM) is dialyzed for 2 h against buffer BE50 (50 mM KCl, 30 mM Hepes pH 7.4, 5 mM MgCl₂, 3% glycerol, 0.5 mM DTT, 0.1 mM AEBSF) and bound in batch to 0.5 ml Poly(U)-Sepharose (GE healthcare) for 90 min at 4°C on a rotating wheel. The sepharose is washed for three times with 5 ml buffer BE50 each, and step-eluted twice in 0.5 ml buffer BE1000 (containing 1 M KCl). Both eluates are pooled and dialyzed for 1 h to buffer BE50 using a dialysis membrane (Millipore 'V' series membranes). The sample is layered on top of a 12 ml linear 5% to 30% (w/w) glycerol gradient adjusted to 50 mM KCl, 30 mM Hepes pH 7.4, 5 mM MgCl₂, 0.5 mM DTT. Centrifugation is performed for 20 h at 37000 rpm at 4°C using a Sorvall TH641 rotor. Standard proteins (bovine serum albumine (BSA), aldolase, catalase, see method section 3) are run in a parallel gradient. Twenty fractions of 0.6 ml are collected sequentially from the top. 0.2 ml of each fraction is precipitated overnight with 1 ml acetone. The precipitates are dissolved in loading buffer, and samples are analyzed by SDS-PAGE followed by silver staining. Protein bands exclusively present in the active fraction (8.6 S, fraction 7 in Figure 3B) are excised and analyzed, together with the liquid fraction 7 by mass spectrometry. For this, the protein samples are reduced with DTT, alkylated by iodine acetamide and trypsinized. Peptides are separated by nano-HPLC chromatography and analyzed on a LTQ ion trap mass spectrometer. Mass data of all peptides are analyzed using the MASCOT-software (Matrix Science).

### 3. Determination of the molecular weight of the HsClp1 complex

To determine the molecular weight of the HsClp1 complex, size exclusion chromatography on a semi-purified kinase active fraction is performed using a Superdex 200 column (Highload 16/60 column, GE healthcare) and buffer BB100 as running buffer. For column calibration, standard proteins are used (thyroglobulin rₛ = 8.5 nm, 19.0 S; catalase rₛ = 5.2 nm, 11.3 S; aldolase rₛ = 4.8 nm, 7.3 S; bovine serum albumin rₛ = 3.6 nm, 4.6 S). The native molecular weight of the HsClp1 protein complex is calculated by the method of Siegel and Monty [52] using the following equation, including the results form the size exclusion chromatography as well as of the glycerol gradient centrifugation:
M = S No (6 π η rₛ) / (1 - v₂ p); M = molecular weight (Da), S = sedimentation coefficient (x 10⁻¹³ sec), No = Avogadro's number (6.022 x 10²³), η = viscosity of the solvent (0.01 g/[cm x sec] for H₂O), rₛ = Stokes Radius (nm x 10⁻⁷), v₂ = partial specific volume (0.73 cm³/g, assumed average for proteins), ρ = density of solvent (1.0 g/ cm³ for H₂O).

### 4. Mammalian cell culture

HeLa cells are cultured at 37 °C, 95% humidity and 5% CO₂. The growth medium is Dulbecco's Modified Eagle's Medium (DMEM) supplemented with 10% fetal bovine serum, 3 mM glutamine, 100 U/ml penicillin and 100 µg/ml streptomycin sulfate (all reagents are from Gibco BRL).

### 5. siRNA-mediated knockdown experiments

To deplete the HsClp1 protein, HeLa cells at 30% confluency are transfected in a 6-well dish with 200 nM of *SMART pool-siRNA* duplexes against HsClp1 (Dharmacon) or with a control siRNA against GFP. Transfection is performed using Lipofectamin 2000 (Invitrogen) according to the manufacturer's instructions. 64 hours post-transfection, cells are lysed in M-PER mammalian protein extraction reagent (Pierce), and samples are dialyzed against buffer BA (100 mM KC1, 30 mM Hepes pH 7.4, 5 mM MgCl_{2,} 10% glycerol, 0.5 mM DTT, 0.1 mM AEBSF). Protein concentrations are then measured by Bradford using the 'BioRad dye reagent concentrate for protein assay' and equalized. Subsequently, the kinase activity of the samples is assessed (see method section 1).

### 6. Cloning of the HsClp1 protein

The Open Reading Frame (ORF) of the HsClp1 protein is cloned by polymerase chain reaction (PCR) using HeLa cDNA as template and KOD High Fidelity Polymerase (Novagen). Primer oligonucleotides (primer 1, 5'- AAA AAG CAG GCT CTA TGG GAG AAG AGG CTA ATG ATG -3', primer 2, 5'- AGA AAG CTG GGT GCT ACT TCA GAT CCA TGA ACC GG -3') are designed to include the flanking ends of the *HsClp1* gene (genebank accession No. NM_006831). For the cloning of the *HsClp1* gene, the Gateway system (Invitrogen) is used. The ORF is recombined into the gcDNA3.1 plasmid, a eukaryotic expression vector encoding a myc-tag 5' terminally to the gene.

The Walker-A mutant versions of HsClp1 are generated by PCR site-directed mutagenesis, exchanging K127A and S128A, and the mutated PCR fragment are recombined into the gcDNA3.1 plasmid.

### 7. Immunoprecipitation experiments

HeLa cells are transfected with gcDNA3.1 plasmids encoding either the myc-tagged wild-type or Walker-A mutant versions (K127A and S128A) of HsClp1 using Lipofectamin 2000 (Invitrogen), or left untransfected. 48 hours post-transfection, cells are treated with lysis buffer (100 mM KC1, 30 mM Hepes pH 7.4, 5 mM MgCl₂, 1% NP40, 10% glycerol, 0.5 mM DTT, 0.1 mM AEBSF) and cell extract is recovered after centrifugation for 10 min at 13000 rpm. 2 µg of α-c-myc antibody (Sigma) are coupled to 40 µl protein A Sepharose 4 FF resin (GE healthcare) by incubating at 4°C for 90 min on a rotating wheel. The resin is washed three times with lysis buffer. Cell extracts are added to the beads, followed by incubation at 4°C for 90 min on a rotating wheel. The resin is washed three times in wash buffer (equals lysis buffer lacking NP40) and the immunoprecipitates are split in two halves. One half is subjected to Western analysis, the other half (20 µl resin) is resuspended in 10 µl wash buffer and 10 µl reaction mixture followed by the kinase assay as described above.

### 8. Purification of recombinant HsClp1 from E.coli

To obtain recombinant HsClp1, the ORF of the hClp1 wild-type or K127A mutant version is recombined into the pDEST15 plasmid (Invitrogen), encoding a N-terminal glutathione *S*-transferase tag, which is then transformed into *E. coli* (Rossetta, strain, Novagen). An overnight culture of 100 ml in LB medium is diluted into 1.2 L of LB medium to OD₆₀₀ₙₘ= 0.1, and after further incubation HsClp1 protein is expressed for 2 h at 37°C by addition of 0.1 mM isopropyl β-D-thiogalactoside (IPTG; Sigma) at an OD₆₀₀ₙₘ= 0.4. Cells are harvested by centrifugation (4000 rpm for 20 min) and the cell pellet is resuspended in 20 ml extraction buffer (50 mM Tris/HCl [pH 8], 100 mM NaCl, 5 mM MgCl₂, 0.1% Tween-20, 1 mM DTT, 0.1 mM AEBSF). The cells are lysed by sonication and centrifuged at 16000 rpm for 30 min. The supernatant is added to 1 ml glutathione-Sepharose FF (GE Healthcare) and incubated on a rotating wheel at 4°C for 2 h. The resin is transferred to a chromatography column (BioRad), and washed with wash buffer (equals extraction buffer lacking Tween-20). The protein is eluted by incubating the resin with 1 ml of elution buffer (equals wash buffer plus 20 mM reduced Glutathione [Sigma]) for 10 min. Finally the protein concentration is measured by Bradford (BioRad Bradford reagent). The total yield of HsClp1 recovered from 1.2 L bacterial culture is 0.3 mg.

### 9. Metal-ion depletion

To test the requirement of divalent metal-ions for the kinase activity of HsClp1, the sample of interest is dialyzed for 1 h against buffer A (100 mM KCl, 30 mM Hepes pH 7.4, 10% glycerol, 0.5 mM DTT, 0.1 mM AEBSF) using dialysis membranes (Millipore 'V' series membrane). EDTA is added at 10 mM, the sample is incubated on ice for 10 min and then dialyzed again for 1 h against buffer A. The sample is then added to reaction mixture R (described above) containing various divalent metal ions at different concentrations, and a kinase assay is performed.

### 10. Immunofluorescence Microscopy

HeLa cells are plated on a glass cover slip in a 6-well dish. At subconfluency, the cells are transfected using Lipofectamin 2000 (Invitrogen) with the gcDNA3.1 plasmid encoding for myc-tagged HsClp1, or left untransfected. 36 hours post-transfection, the cells are rinsed twice with PBS (137 mM NaCl, 2.7 mM KCl, 4.3 mM
Na₂HPO₄.7H₂O, 1.4 mM KH₂PO₄, pH7.3), fixed with 3% para-formaldehyde in PBS for 20 min at RT, washed twice in PBS and permeabilized with 0.3% Triton X-100 for 5 min on ice. Cells are washed again three times with PBS and blocked with 3% BSA (Sigma) in PBS for 1 h. The cells are then incubated with α-c-myc antibody (Sigma, 0.8 ng/µl, in 3% BSA/PBS) at RT for 45 min in a humid chamber, washed three times with 0.1 % BSA/PBS and incubated with Alexa 488 goat α-rabbit (Molecular Probes, 1:1000, in 3% BSA/PBS). Cells are washed for three times with 0.1% BSA/PBS, incubated with DAPI (4',6-diamidino-2-phenylindole) solution (1 µg/ml) for 5 min and washed three times with deionized H₂O. The cover slips are finally mounted onto slides in Vectashield mounting medium (Vector). For microscopy, a Zeiss Axioplan2 imaging microscope and a Coolsnap HQ CCD digital camera are used, and images are processed using and Adobe Photoshop Software.

### Example 1

### Identification of HsClp1 as the 'siRNA kinase'

### a) Purification of an siRNA-kinase activity from human cytoplasmic extracts

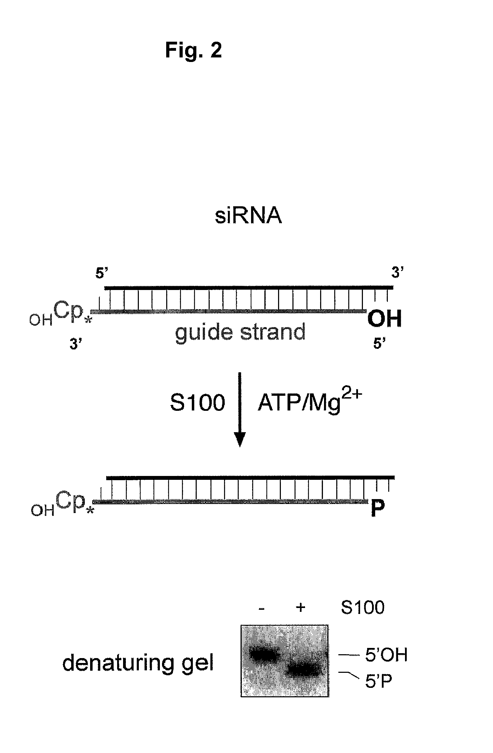

In order to identify the enzyme activity that phosphorylates siRNAs in human cells, an assay is established that enables to monitor the 5' phosphorylation status of the guide strand within a siRNA duplex [19]. The guide strand, containing a 5'-hydroxyl group, is 3'-end-labeled by ligation to Cytidine 3',5'-bis [α-³²P] phosphate, and the terminal phosphate group is removed by treatment with alkaline phosphatase. Then the labeled RNA is annealed to a complementary strand. After a few minutes of incubation in HeLa cytoplasmic (S100) extract in the presence of ATP, a migration shift during electrophoresis of the siRNA is indicative of 5'-end phosphorylation of the guide strand by a kinase activity (Figure 2). The main goal is to purify the kinase from HeLa S 100 extracts using classical chromatography. The inventors establish eight purification steps throughout which a single kinase activity is followed. A scheme of the purification flow is depicted in Figure 3A. Fractions of the final purification step (glycerol gradient centrifugation) are subjected to SDS-PAGE followed by silver staining (Figure 3B). A ~45 kDa polypeptide is found to co-fractionate with the kinase activity (Figure 3B, fraction 7). This band is excised from the gel and analyzed by mass spectrometry. Polypeptides corresponding to the HsClp1 protein (TREMBL accession No. Q92989) are found to be predominant in this band. Interestingly, the HsSen endonuclease subunits HsSen2 and HsSen54 are also identified on the silver-stained gel in fraction 7 (Figure 3B), and the additional detection of the HsSen15 and HsSen34 protein by mass spectrometric analysis on the liquid sample demonstrated the presence of all known subunits HsSen complex. This is in accordance with the observation that HsClp1 associates with the HsSen complex in cell extracts.

### b) Evaluation of HsClp1 as the enzyme that phosphorylates siRNAs

As the HsClp1 protein contains an ATP/GTP binding motif (Walker A motif) [30], it is suspected to represent the kinase. The inventors set out to validate this candidate protein using three different approaches. Firstly, the inventors deplete HeLa cells of HsClp1 by transfection with siRNAs complementary to its coding sequence. The extracts obtained are then assayed for kinase activity in a time-course experiment. As shown in Figure 4A, such extracts show a markedly slower kinetics of 5'-end phosphorylation when compared to extracts derived from cells that are treated with a control GFP-siRNA. In a second approach, the coding sequence of HsClp1 (1278 nt) is amplified from HeLa cDNA and cloned as a myc-tagged fusion into an expression plasmid. The inventors also generate a Walker A-motif mutant version of the kinase (Lysine 127 → Alanine), since this motif is most likely to be essential for the phosphorylation reaction [53]. HeLa cells are transfected with the plasmids followed by immunoprecipitation of extracts using α-myc antiserum. As shown in Figure 4B, immunoprecipitates containing the wild-type but not the mutant version of HsClp1-protein show efficient 5'-end phosphorylation, demonstrating a direct link between the protein and the kinase activity. Thirdly, the inventors assay the potential kinase activity of the recombinant HsClp1-protein. For this, the inventors express HsClp1 as glutathione *S*-transferase fusion in *Escherichia coli* and further purify it using affinity chromatography on glutathione sepharose. The recombinant protein is purified to near-homogeneity as confirmed by Coomassie staining (Figure 4C, upper panel). The eluted recombinant HsClp1 protein on its own shows siRNA-kinase activity, and it is therefore concluded that it is the sole catalyst in the kinase reaction (Figure 4C, lower panel). Taken together, these three lines of evidence demonstrate that HsClp1 indeed represents the 'siRNA kinase'.

### Example 2

### HsClp1 is part of a protein complex

Next it is investigated, whether HsClp1 exists as a monomeric protein or is part of a protein complex in cell extracts. The sedimentation coefficient of HsClp1, determined by glycerol gradient centrifugation at the final purification step, is 8.6 S (Figure 3B). The stokes radius of HsClp1, measured by gel exclusion chromatography using a Superdex 200 column, is 5.6 - 6.4 nm (Figure 3C). The molecular weight of the kinase derived from these values using the equation of Siegel and Monty is 200 - 230 kDa [52]. These data suggest that HsClp1 is part of a protein complex in cell extracts, presumably as part of the HsSen complex as mentioned above.

### Example 3

### HsClp1 discriminates between ssRNA and ssDNA

It is then assessed whether HsClp1 exclusively phosphorylates duplex siRNAs or in addition uses other nucleic acids as substrates. This is tested by incubating various single- and double-stranded RNA and DNA molecules, or RNA/DNA hybrids either with 'endogenous' HsClp1 purified form HeLa extracts (purified HsClp1, Figure 5A-C), or with recombinant HsClp1 protein (see Figure 5D-F) to ascertain that its biochemical activities reflect the ones of the 'endogenous' purified protein (Figure 5D-F). In parallel, all substrates are subjected to phosphorylation by recombinant T4 PNK to compare substrate specificity with HsClp1. A DNA 'guide strand' is 3'-end-labeled with α-³²P-cordycepin 5' triphosphate and annealed to a complementary DNA oligonucleotide. As shown in Figure 5A and 5D (right panel), this dsDNA is phosphorylated at the 5'-end by both, purified and recombinant HsClp1 proteins, with a similar kinetics as for dsRNA (Figure 5A and 5D, left panel). Then phosphorylation of RNA/DNA hybrid duplexes by HsClp1 is tested. In contrast to the efficient 5'-end phosphorylation of a guide RNA within a RNA/DNA hybrid duplex, the 5'-end of a guide DNA strand within RNA/DNA is only a very poor substrate for HsClp1, if any at all (Figure 5B and 5E). These data show that HsClp1 not only efficiently phosphorylates dsRNA, but also dsDNA and RNA within a RNA/DNA duplex.

Single-stranded guide RNA (ssRNA) is also a good substrate for HsClp1 (Figure 5C and 5F). Interestingly, ssDNA is not phosphorylated at all, despite being a good substrate for T4 polynucleotide kinase (Figure 5C and 5F). In order to determine, to which extent deoxy-ribonucleotides are substrates for the kinase, the inventors design a ssRNA in which the 5' outermost nucleotide is replaced with 2' deoxythymidine (dT). Even after 2 h incubation time with HsClp1 protein, such an oligonucleotide is not detectably phosphorylated. These data suggest that, even though HsClp1 does not discriminate between 2' deoxy- and 2' hydroxyl-ribonucleotides when presented in duplexes, discrimination occurs at the single-stranded level. In addition, these analyses altogether also confirm that the substrate specificity of 'endogenous' HsClp1 is fully reflected by the recombinant protein.

It is next tested whether the HsClp1 protein discriminates between different overhangs in siRNAs using HeLa S100 extracts. Phosphorylation of 2nt 5'-overhangs and blunt ends occurred at the same efficiency as the canonical siRNA 2nt 3'-overhangs (Figure 6A). In addition, the kinase activity is not depending on the nucleotide composition of the 2nt 3'-overhang, and even phosphorylates siRNAs containing 2nt deoxy-thymidine overhangs (Figure 6B). The 5' position of siRNAs is phosphorylated irrespective of the nucleotide composition (Figure 6C). Surprisingly, although the kinase cannot phosphorylate a deoxythymidine at the 5' end of single-stranded RNA (Figure 5C and 5F), it is able to phosphorylate it when present in a dsRNA (Figure 6C).

### Example 4

### The divalent metal ion and ATP/GTP requirements for the kinase activity of HsClp1

The biochemical characterization of HsClp1 is extended and the requirement of the kinase activity for divalent metal ions analyzed. A semi-purified HsClp1 fraction from HeLa extracts, or the recombinant HsClp1 (see Figure 4C) are dialyzed against a buffer lacking metal ions, then treated with EDTA to complex residual metal ions, and finally dialyzed against a buffer devoid of metal ions to remove Me²⁺-EDTA complexes. The kinase activity of HsClp1 on siRNA is then assessed in reaction mixtures supplemented with various divalent metal ions at different concentrations. As shown in Figure 7A, metals such as Ca²⁺, Co²⁺, Mn²⁺ and Mg²⁺ are very efficient in stimulating 'endogenous' kinase activity in a broad concentration range (1- 20 mM), whereas Fe²⁺, Ni²⁺ and Zn²⁺ stimulate the kinase activity at the narrow concentration window of 2 mM. Cu²⁺ is not used as a co-factor by the kinase at all. In contrast, recombinant HsClp1 only shows kinase activity in the presence of Mg²⁺ and Mn²⁺at 2-5 mM, and Ni²⁺ at 2 mM, but not with the other metal ions (Figure 7B).

It is next tested whether 'endogenous' semi-purified HsClp1 or recombinant HsClp1 exclusively uses ATP as a phosphodonor or is able to phosphorylate siRNAs using GTP. Reaction mixtures are supplemented with ATP or GTP at different concentrations and the kinase activity is assessed. Whereas the recombinant HsClp1 protein can only phosphorylate siRNAs using ATP, the 'endogenous' kinase shows siRNA phosphorylation in the presence of both, ATP and GTP (Figure 8).

Taken together, these data show that various metal ions can be used to stimulate the kinase activity of 'endogenous' as well as recombinant HsClp1, and that GTP, in addition to ATP, represents a co-factor for 'endogenous' HsClp1 activity. However, the data also indicate a discrepancy between 'endogenous' and recombinant HsClp1 in the usage of co-factors, suggesting that the conformation or binding partners present in cell extracts may contribute to the mechanism of nucleic acid phosphorylation.

### Example 5

### The ion and pH requirements of HsClp1

The buffer conditions under which HsClp1 show kinase activity is analyzed. The inventors dialyze HeLa S100 extract against buffer containing 10 mM KCl up to 2 M KCl, or buffer containing either 50 mM MES (Morpholineethanesulfonic acid) buffer, pH 6.0 or 50 mM CHES (N-Cyclohexyl-2-aminoethanesulfonic acid) buffer, pH 9.0, followed by a kinase assay. Efficient kinase activity is observed in a range of 10 - 200 mM KCl, and is equally detected at pH 6.0 or pH 9.0 (data not shown), suggesting that the biochemical activity of HsClp1 can be recovered using corresponding buffer compositions.

### Example 6

### HsClp1 localizes predominantly to the nucleus

To analyze the subcellular localization of HsClp1, the myc-tagged protein is transiently overexpressed in HeLa cells. Cells are fixed with para-formaldehyde and immunostained using polyclonal α-myc antiserum. HsClp1 is predominantly localized to the nucleus (Figure 9). In addition, a small pool of the kinase is diffusely distributed across the cytoplasm. Interestingly, the nuclear staining shows a pattern characteristic for proteins found in specialized subnuclear structures called nuclear speckles (Figure 9, arrows) [54].

### Example 7

### Generating a Clp1 knock-out mouse and cell lines

To target Clp1 genomic DNA, a targeting vector is constructed containing Clp1 genomic DNA (derived from the BAC clone RP23-387F9, BACPAC Resources) comprising its exons 1-3. The exon 2 of Clp1 is a suitable target to be deleted, as it contains the ATP/GTP binding site, thus resulting in a non-functional protein. Such a exon 2 targeting vector comprises the upstream part of exon 1 followed by exon 1 (recombination site 1), followed by a LoxP site integrated into the upstream part of exon 2. Downstream of exon 2, a Neomycin resistance cassette flanked by a FRT (for Flp mediated recombination)-LoxP (for Cre-mediated recombination) site on either end is integrated, followed by a genomic stretch upstream of exon 3 (recombination site 2). This vector is then linearized by a restriction site downstream of the Neomycin cassette and electroporated into mouse ES cells. ES cells which have undergone homologous recombination between recombination site 1 and site 2 (to be checked for by PCR and Southern blotting) are selected for by cultivating them for several days in the presence of Neomycin, and are thereafter injected into early-stage mouse embryos. The embryos are implanted into the uterus of a female mouse and are allowed to develop into mouse pups. The obtained mouse chimera are crossbred with a wild-type C57BL/6 mouse strain to obtain heterozygous Flox mice, that are crossed with Flp recombinase transgenic mice to remove the Neomycin resistance cassette between the FRT sites. The resulting strain contains the exon 2 flanked by LoxP sites, and by crossing it into tissue-specific Cre recombinase transgenic mice and after crossbreeding to obtain homozygous floxed Clp1 mice, the Clp1 exon 2 can be deleted in a tissue-specific manner.

Clp1 knock-out cell lines, preferentially mouse embryonic fibroblasts (MEF), can be derived from mice in which Clp1 exon 2 is flanked by LoxP sites by conventional methods as used in [55]. Alternatively, ES cells can be derived from such mice to obtain Clp1 knock-out cell lines. Cre-mediated deletion of Clp1 can then be achieved by infection with a high titer of Cre-adenovirus in tissue culture.

### Example 8

### Assay to screen for small molecule agonists or antagonists of HsClpl's kinase activity

To perform large scale screens for small molecule agonists and antagonists of the kinase activity of HsClp1, the following assay may be conducted. Biotinylated siRNAs (as described in [11], either with a 2 nt, 3' overhang or blunt-ended) are allowed to bind to Strepatvidin-coated microtiter plates (for example SigmaScreen, Sigma). Recombinant active hClp1 protein (such as obtained in method section 8) is transferred to the wells together with [γ-³²P]ATP and the small molecule candidate as part of a library. After incubation to allow siRNA phosphorylation, followed by extensive washes, the amount of incorporation of [γ-³²P] into siRNAs, detected by conventional autoradiography, serves as a measure whether a small molecule exhibits agonistic or antagonistic properties towards hClpl's kinase, depending on whether the incorporation of [γ-³²P] is more or less, respectively, in comparison to a control well lacking small molecules. Instead of [γ-³²P]ATP, fluorescently labeled ATP or ATP-analogs (Jena Bioscience) may be used, coupled with photometric measurements as a readout for the kinase activity. Alternatively, in such a microtiter-plate based assay, the coupled biotinylated siRNAs may be incubated with ATP together recombinant HsClp1 and a small molecule candidate. After extensive washes, phosphorylation efficiency may be detected by recombinant antibody (MorphoSys) that differentiates between the 5' phosphorylated or unphosphorylated form of a siRNA. After washes, the binding efficiency of a fluorescently labeled secondary antibody, detected by photometric methods, is a measurement for agonistic or antagonistic activities of small molecule on hClp1's kinase activity.

### References

1. Meister, G., and Tuschl, T. (2004). Mechanisms of gene silencing by double-stranded RNA. Nature 431, 343-349.
2. Tomari, Y., and Zamore, P.D. (2005). Perspective: machines for RNAi. Genes Dev 19,517-529*.*
3. Fire, A., Xu, S., Montgomery, M.K., Kostas, S.A., Driver, S.E., and Mello, C.C. (1998). Potent and specific genetic interference by double-stranded RNA in Caenorhabditis elegans. Nature 391, 806-811.
4. Hannon, G.J., and Rossi, J.J. (2004). Unlocking the potential of the human genome with RNA interference. Nature 431, 371-378.
5. Bernstein, E., Caudy, A.A., Hammond, S.M., and Hannon, G.J. (2001). Role for a bidentate ribonuclease in the initiation step of RNA interference. Nature 409, 363-366.
6. Elbashir, S.M., Lendeckel, W., and Tuschl, T. (2001). RNA interference is mediated by 21 and 22 nt RNAs. Genes Dev 15, 188-200.
7. Bartel, D.P. (2004). MicroRNAs: genomics, biogenesis, mechanism, and function. Cell 116, 281-297.
8. Ambros, V. (2004). The functions of animal microRNAs. Nature 431, 350-355.
9. Hammond, S.M., Bernstein, E., Beach, D., and Hannon, G.J. (2000). An RNA-directed nuclease mediates post-transcriptional gene silencing in Drosophila cells. Nature 404, 293-296.
10. Hutvagner, G., and Zamore, P.D. (2002). A microRNA in a multiple-turnover RNAi enzyme complex. Science 297, 2056-2060.
11. Martinez, J., Patkaniowska, A., Urlaub, H., Lührmann, R., and Tuschl, T. (2002). Single-stranded antisense siRNAs guide target RNA cleavage in RNAi. Cell 110, 563-574.
12. Schwarz, D.S., Hutvagner, G., Du, T., Xu, Z., Aronin, N., and Zamore, P.D. (2003). Asymmetry in the assembly of the RNAi enzyme complex. Cell 115, 199-208.
13. Khvorova, A., Reynolds, A., and Jayasena, S.D. (2003). Functional siRNAs and miRNAs exhibit strand bias. Cell 115, 209-216.
14. Matranga, C., Tomari, Y., Shin, C., Bartel, D.P., and Zamore, P.D. (2005). Passenger-strand cleavage facilitates assembly of siRNA into Ago2-containing RNAi enzyme complexes. Cell 123, 607-620.
15. Rand, T.A., Petersen, S., Du, F., and Wang, X. (2005). Argonaute2 cleaves the anti-guide strand of siRNA during RISC activation. Cell 123, 621-629.
16. Leuschner, P.J.F., Ameres, S.L., Kueng, S., and Martinez, J. (2006). Cleavage of the siRNA passenger strand during RISC assembly in human cells. EMBO Rep in press*.*
17. Liu, J., Carmell, M.A., Rivas, F.V., Marsden, C.G., Thomson, J.M., Song, J.J., Hammond, S.M., Joshua-Tor, L., and Hannon, G.J. (2004). Argonaute2 Is the Catalytic Engine of Mammalian RNAi. Science 305, 1437-1441.
18. Rivas, F.V., Tolia, N.H., Song, J.J., Aragon, J.P., Liu, J., Hannon, G.J., and Joshua-Tor, L. (2005). Purified Argonaute2 and an siRNA form recombinant human RISC. Nat Struct Mol Biol 12, 340-349.
19. Nykänen, A., Haley, B., and Zamore, P.D. (2001). ATP requirements and small interfering RNA structure in the RNA interference pathway. Cell 107, 309-321.
20. Chiu, Y.L., and Rana, T.M. (2002). RNAi in human cells. Basic structural and functional features of small interfering RNA. Mol Cell 10, 549-561.
21. Schwarz, D.S., Hutvagner, G., Haley, B., and Zamore, P.D. (2002). Evidence that siRNAs function as guides, not primers, in the Drosophila and human RNAi pathways. Mol Cell 10, 537-548.
22. Pham, J.W., and Sontheimer, E.J. (2005). Molecular requirements for RNA-induced silencing complex assembly in the Drosophila RNA interference pathway. J Biol Chem 280, 39278-39283.
23. Pellino, J.L., Jaskiewicz, L., Filipowicz, W., and Sontheimer, E.J. (2005). ATP modulates siRNA interactions with an endogenous human Dicer complex. Rna 11, 1719-1724.
24. Elbashir, S.M., Martinez, J., Patkaniowska, A., Lendeckel, W., and Tuschl, T. (2001). Functional anatomy of siRNAs for mediating efficient RNAi in Drosophila melanogaster embryo lysate. Embo J 20, 6877-6888.
25. Shuman, S., and Hurwitz, J. (1979). 5'-Hydroxyl polyribonucleotide kinase from HeLa cell nuclei. J Biol Chem 254, 10396-10404.
26. Tanabe, S., Bohlander, S.K., Vignon, C.V., Espinosa, R., Zhao, N., Strissel, P.L., Zeleznik-Le, N.J., and Rowley, J.D. (1996). AF10 is split by MLL and HEAB, a human homologue to a putative Caenorhabditis elegans ATP/GTP-binding protein in an invins (10;11)(p12;q23q12). Blood 88, 3535-3545.
27. Walker, J.E., Saraste, M., Runswick, M.J., and Gay, N.J. (1982). Distantly related sequences in the α- and β-subunits of ATP synthase, myosin, kinases and other ATP-requiring enzymes and a common nucleotide binding fold. EMBO J 1, 945-951.
28. Saraste, M., Sibbald, P.R., and Wittinghofer, A. (1990). The P-loop - a common motif in ATP- and GTP-binding proteins. Trends Biochem Sci 15, 430-434.
29. Minvielle-Sebastia, L., Preker, P.J., Wiederkehr, T., Strahm, Y., and Keller, W. (1997). The major yeast poly(A)-binding protein is associated with cleavage factor IA and functions in premessenger RNA 3'-end formation. Proc Natl Acad Sci U S A 94, 7897-7902.
30. deVries, H., Ruegsegger, U., Hubner, W., Friedlein, A., Langen, H., and Keller, W. (2000). Human pre-mRNA cleavage factor II(m) contains homologs of yeast proteins and bridges two other cleavage factors. EMBO J 19, 5895-5904.
31. Wahle, E., and Ruegsegger, U. (1999). 3'-end processing of pre-mRNA in eukaryotes. FEMS Microbiol Rev 23, 277-295.
32. Zhao, J., Hyman, L., and Moore, C. (1999). Formation of mRNA 3' ends in eukaryotes: mechanism, regulation, and interrelationships with other steps in mRNA synthesis. Microbiol Mol Biol Rev 63, 405-445.
33. Abelson, J., Trotta, C.R., and Li , H. (1998). tRNA splicing. J Biol Chem 273, 12685-12688.
34. Paushkin, S.V., Patel, M., Furia, B.S., Peltz, S.W., and Trotta, C.R. (2004). Identification of a human endonuclease complex reveals a link between tRNA splicing and pre-mRNA 3' end formation. Cell 117, 311-321.
35. Richardson, C.C. (1965). Phosphorylation of nucleic acid by an enzyme from T4 bacteriophage-infected Escherichia coli. Proc Natl Acad Sci U S A 54, 158-165.
36. van Houten, V., Denkers, F., van Dijk, M., van den Brekel, M., and Brakenhoff, R. (1998). Labeling efficiency of oligonucleotides by T4 polynucleotide kinase depends on 5'-nucleotide. Anal Biochem 265, 386-389.
37. Sambrook, J., Fritsch, E., and Maniatis, T. (1989). Molecular Cloning, 2nd Edition (Plainview, NY: Cold Spring Harbor Laboratory Press).
38. Winnacker, E.L. (1987). From genes to clones : introduction to gene technology. VCH publishers, New York, 451-481.
39. Braselmann, S., Graninger, P., and Busslinger, M. (1993). A selective transcriptional induction system for mammalian cells based on Gal4-estrogen receptor fusion proteins. Proc Natl Acad Sci U S A 90, 1657-1661.
40. Kohli, M., Rago, C., Lengauer, C., Kinzler, K.W., and Vogelstein, B. (2004). Facile methods for generating human somatic cell gene knockouts using recombinant adeno-associated viruses. Nucleic Acids Res 32, p. e3.
41. Hirata, R., Chamberlain, J., Dong, R., and Russell, D.W. (2002). Targeted transgene insertion into human chromosomes by adeno-associated virus vectors. Nat Biotechnol 20, 735-738.
42. Samuels, Y., Diaz, L.A., Jr., Schmidt-Kittler, O., Cummins, J.M., Delong, L., Cheong, I., Rago, C., Huso, D.L., Lengauer, C., Kinzler, K.W., Vogelstein, B., and Velculescu, V.E. (2005). Mutant PIK3CA promotes cell growth and invasion of human cancer cells. Cancer Cell 7, 561-573.
43. Nagy, A. (2000). Cre recombinase: The universal reagent for genome tailoring. Genesis 26, 99-109.
44. Praitis, V., Casey, E., Collar, D., and Austin, J. (2001). Creation of low-copy integrated transgenic lines in Caenorhabditis elegans. Genetics 157, 1217-1226.
45. Ashburner, M., Golic, K.G., and Hawley, R.S. (2005). Drosophila - a laboratory handbook. Cold Spring Harbor Laboratory Press 2nd editi*on.*
46. Spradling, A.C., and Rubin, G.M. (1982). Transposition of cloned P elements into Drosophila germ line chromosomes. Science 218, 341-347.
47. Brummelkamp, T.R., Bernards, R., and Agami, R. (2002). A system for stable expression of short interfering RNAs in mammalian cells. Science 296, 550-553.
48. Sui, G., Soohoo, C., Affar el, B., Gay, F., Shi, Y., and Forrester, W.C. (2002). A DNA vector-based RNAi technology to suppress gene expression in mammalian cells. Proc Natl Acad Sci U S A 99, 5515-5520.
49. Xia, H., Mao, Q., Paulson, H.L., and Davidson, B.L. (2002). siRNA-mediated gene silencing in vitro and in vivo. Nat Biotechnol 20, 1006-1010.
50. Ryther, R.C., Flynt, A.S., Phillips, J.A., 3rd, and Patton, J.G. (2005). siRNA therapeutics: big potential from small RNAs. Gene Ther 12, 5-11.
51. Dignam, J.D., Lebovitz, R.M., and Roeder, R.G. (1983). Accurate transcription initiation by RNA polymerase II in a soluble extract from isolated mammalian nuclei. Nucleic Acids Res 11, 1475-1489.
52. Siegel, L.M., and Monty, K.J. (1966). Determination of molecular weights and frictional ratio of proteins in impure systems by use of gel filtration and density gradient centrifugation. Biochim Biophys Acta 112, 346-362.
53. Wang, L.K., and Shuman, S. (2001). Domain structure and mutational analysis of T4 polynucleotide kinase. J Biol Chem 276, 26868-26874.
54. Lamond, A.I., and Spector, D.L. (2003). Nuclear speckles: a model for nuclear organelles. Nat Rev Mol Cell Biol 4, 605-612.
55. Dodge, E.J., Okano, M., Dick, F., Tsujimoto, N., Chen, T., Wang, S.L., ueda, Y., Dyson, N., and Li, E. (2005). Inactivation of Dnmt3b in mouse embryonic fibroblasts results in DNA hypomethylation, chromosomal instability, and spontaneous immortalization. J Biol Chem 280, 17986-17991.

## Claims

1. The use of a Clp1 molecule as an RNA kinase.

2. The use of claim 1 for the transfer of the γ-phosphate of ATP to the 5' end of ssRNA, dsRNA or the RNA strand of RNA/DNA hybrids.

3. The use of claim 1 or 2, wherein said Clp1 is recombinant.

4. The use of claim 1, wherein said Clp1 is human Clp1 (HsClp1) with the amino acid sequence as set forth in SEQ ID NO: 2, or with the amino acid sequence encoded by a polynucleotide which hybridizes under stringent conditions to a polynucleotide having a nucleotide sequence as set forth in SEQ ID NO:1 or to the region encoding HsClp1 contained therein, or a variant or fragment thereof with said RNA kinase activity.

5. The use of any one of claims 1 to 4, wherein said Clp1 molecule is a component of a kit.

6. A kit containing
a. a recombinant Clp1 with said RNA kinase activity;
b. γ-ATP; and
c. a reaction buffer containing buffer components and one or more metal ions selected from Mg²⁺, Mn²⁺, Ni² or mixtures thereof, for use in a final concentration range of ca. 1 - 10 mM.

7. The kit of claim 6, wherein said concentration range of the metal ion 2 - 5 mM.

8. The kit of claim 6 or 7, whererin γ-ATP is present in radioactively labeled form.

9. The kit of claim 8, wherein γ-ATP is present as [γ-³² P]ATP, [γ-³³P]ATP, [γ-¹⁸O]ATP or [γ-³⁵S]ATP.

10. The kit of any one of claims 6 to 9, wherein said Clp1 is recombinant human Clp1 as defined in claim 4.

11. The use of any one of claims 1 to 5, wherein said Clp1 molecule is exogenously expressed in an animal cell to exhibit its kinase activity in said cell..

12. The use of claim 11, wherein said animal cell is a mammalian cell.

13. The use of claim 12, wherein said cell is derived from a cell line.

14. An animal cell line genetically engineered to express Clp1.

15. The cell line of claim 13 which does not endogenously express Clp1.

16. The cell line of claim 14 or 15, which, in addition, expresses an siRNA molecule.

17. The cell line of claim 16, wherein said siRNA molecule is controlled by the same promoter as said Clp1 molecule.

18. A Clp1 transgenic non-human animal.

19. The transgenic animal of claim 18 which is a mouse.

20. A Clp1 knock-out mouse.

21. A pharmaceutical composition comprising a DNA encoding a therapeutic siRNA directed against a disease-associated gene of interest and a DNA encoding a human Clp1 molecule with said RNA kinase activity.
